# EUROPEAN PATENT APPLICATION

(11) **EP 2 556 842 A1**
(43) Date of publication of application: **13.02.2013**
(21) Application number: 11382276.1
(22) Date of filing: 11.08.2011
(51) Int. Cl.: A61L 24/10

(54) **Composition in the form of film comprising fibrinogen and a fibrinogen activator and the applications thereof**

(71) Applicant: Bioftalmik, S.L., 48160 Derio (Vizcaya) (ES)
(72) Inventor: Vega Chaparro, Sandra Clarissa, E-48160 Derio - Vizcaya (ES); Suarez Cortés, Tatiana, E-48160 Derio - Vizcaya (ES); Duran de la Colina, Juan Antonio, E-48006 Bilbao - Vizcaya (ES)
(74) Representative: ABG Patentes, S.L.

(57) **Abstract**

The composition in the form of solid film comprises a layer (A) comprising a fibrinogen activator and a biocompatible material and a layer (B) comprising fibrinogen and a biocompatible material, wherein the fibrinogen activator and the fibrinogen are not present simultaneously in one and the same layer. The invention also describes a composition in the form of film comprising a compressed mixture of a lyophilisate comprising a fibrinogen activator and a lyophilisate comprising fibrinogen. Said compositions can be used in the preparation of fibrin adhesives and in hemostasis, tissue adhesion and wound healing.

## Description

### Field of the Invention

The present invention generally relates to a composition in the form of solid film comprising fibrinogen and a fibrinogen activator and the applications thereof. In particular, the invention relates, on one hand, to a composition in the form of film comprising a layer comprising a fibrinogen activator and a layer comprising fibrinogen, wherein the fibrinogen activator and the fibrinogen are not present simultaneously in one and the same layer, and on the other hand, to a composition in the form of film comprising a compressed mixture of a lyophilisate comprising a fibrinogen activator and a lyophilisate comprising fibrinogen. The invention also relates to obtaining said compositions and to the applications thereof, particularly to the use of said compositions in the preparation of fibrin adhesives and in hemostasis, tissue adhesion and wound healing.

### Background of the Invention

The use of blood substances for closing wounds using the hemostasis dates from the year 1909 (Donkerwolcke, M. et al. 1998. Biomaterials, 19:1461-1466). Specifically, fibrin adhesive itself was described for the first time in 1940 (M1orikawa, T. 2001. The American Journal of Surgery, 182:29-35) and the combination of fibrinogen and thrombin was clinically used for the first time in 1944 to improve the adhesion of skin allografts to soldiers with burns. Their low adhesion qualities and the risk of transmitting infections discouraged their use. However, in the year 1972 the fibrin made a comeback as adhesive for joining human nerves. Its marketing started in the 80's in Europe (MacGillivray, E.T. 2003. Journal of Cardiovascular Surgery, 18: 480-485).

The tissue adhesion and hemostasis with fibrin have been used for stopping large surface hemorrhages, tissue repair and/or adhesion in parenchymatous organs, closing of wounds in plastic surgery, connective tissue adhesion or assuring suture and hemostasis after many surgical operations, among others (Silver, F.H. et al. 1995. Biomaterials, 16: 891-903). The essential advantage in comparison with other methods (e.g. adhesion with synthetic tissue adhesives) is the use of substances from the body itself showing a good compatibility and positively influencing wound healing.

In tissue adhesion the final phase of the clotting is completed: the fibrinogen is contacted with thrombin and calcium ions on the surface to be adhered. The active thrombin is a serine-protease which hydrolyzes the fibrinogen generating fibrin monomers. These monomers aggregate spontaneously forming a relatively weak fibrin clot and at the same time, by means of thrombin and calcium ions, the factor XIII transglutaminase which introduces covalent bonds between the fibrin monomers is activated such that the clot stabilizes by means of reticulation of the monomers thereof. A high resistance of the clot against fibrinolysis can be achieved if desired by means of adding a plasmin inhibitor to the fibrinogen concentrate.

For the tissue adhesion with fibrin adhesives, there is provided, on one hand, with commercial preparations and, on the other hand, with the fibrin adhesives prepared by the user at the time of application from blood samples such as those described in the international patent application WO 86/01814.

The commercially available fibrin adhesives which include Tissucol^{®}, are adhesives of two components, a first component comprising fibrinogen, factor XIII and aprotinin, and a second component comprising thrombin and calcium ions. Both components are presented either in the form of frozen solutions in preloaded syringes or in the form of lyophilisates. Their mechanism of action consists of the formation of an adherent mixture reproducing the final stage of the blood clotting cascade such that the mixture of the fibrinogen with the thrombin forms a fibrin clot wherein the fibrinogen molecule is dissociated due to the action of the thrombin to form fibrin monomers which polymerize spontaneously forming a three-dimensional fibrin network which is fundamentally retained by means of covalent bonds. A drawback of these commercially available fibrin adhesives lies in the high cost and the long preparation required for the fibrin adhesive until reaching the ideal conditions for its immediate application since both unfreezing and heating to the temperature of application, and reconstituting the lyophilisates conditioned by the high concentration of fibrinogen (approximately 6-10% by weight with respect to the total weight), require a relatively long time period. Furthermore, the relatively short preservation time of the components of the fibrin adhesive once it is ready for use (approximately 4 hours), make up a significant drawback in the commercially available fibrin adhesives since, in event that unpredicted delays arise between the preparation of the fibrin adhesive and its application, the fibrin adhesive can become useless or lose its efficiency, such as mentioned in the European patent EP 0 554 570 B1. Additionally, with said commercially available fibrin adhesives it is not possible to prepare the amount of fibrin adhesive needed for a particular application since all the product (components) contained in the vials or preloaded syringes is unfrozen and/or reconstituted such that the fibrin adhesive which is not used must be discarded, therefore in the minimally invasive surgeries (for example, some surgeries in the ophthalmology field) most of the fibrin adhesive is lost, this translates into in a significant economic expenses since if it is not discarded and is used to treat other patients, the safety of said patients would be compromised since the risk of cross-contamination cannot be ruled out.

For the purpose of overcoming the mentioned drawbacks, many formulations of fibrin adhesive in solid form, for example, hemostatic, bioabsorbable, fibrous, solid compositions comprising a bioabsorbable polymer and a hemostatic compound comprising thrombin and fibrinogen (WO 99/56798), hemostatic bands in the form of sandwich comprising one layer of thrombin between two layers of fibrinogen (US 6,762,336), solid compositions essentially made up of a vehicle with particular physical characteristics and solid thrombin and fibrinogen distributed uniformly on said vehicle (WO 02/058749), and fibrin adhesives based on mixtures of microparticles comprising thrombin and on microparticles comprising fibrinogen (WO 2010/136588, WO 2010/136818 and WO 2010/136819), have been developed.

Although some of said formulations solve some of the drawbacks mentioned above, there is still the need of developing alternative formulations providing solution, if not to the entirety, to a good part of said drawbacks in a single product. Advantageously, said new formulations of fibrin adhesive or fibrin adhesive precursors must be in a ready-to-use form or wherein the activation time of the bioadhesive is short (equal to or less than five minutes), they must be stable for long time in standard storage conditions (room temperature, protected from humidity and light) without requiring special storage installations or devices, for example, freezers, thermostatic baths, etc., they must assure the stability of the active components (thrombin and fibrinogen), they must allow activating the adhesive which will advantageously present the fibrin dimensions and doses necessary for a particular application resulting in the economic savings that it entails and the elimination of the risk of cross-contaminations.

### Summary of the Invention

The inventors have surprisingly discovered that, compositions in the form of film comprising a layer comprising a fibrinogen activator such as thrombin, and a layer comprising fibrinogen, wherein the fibrinogen activator and the fibrinogen are not present simultaneously in one and the same layer, or alternatively, a compressed mixture of a lyophilisate comprising a fibrinogen activator and a lyophilisate comprising fibrinogen, allow generating *in situ* a fibrin adhesive and overcome the drawbacks and limitations of the state of the art mentioned above.

The compositions provided by this invention solve said drawbacks and limitations since, on one hand, they are in ready-to-use form requiring a preliminary hydration and activation demanding a short time period (between approximately one and three minutes) which assures a fast availability of the product for use by reducing the time period required to unfreeze or dissolve the components of the fibrin adhesive. The active components (fibrinogen activator and fibrinogen) of the compositions provided by this invention are kept functional for long storage time periods due to the presentation form (a dry solid) in which they are found in the form of bilayer film of the active ingredients in combination with a biocompatible material or in the form of a single layer from compressed lyophilisates. The compositions of the present invention also allow using the optimum amount needed of said active components as a result of the single-dose presentation in many sizes to obtain the desired effect preventing (or minimizing) the need to discard large excesses from said active components when a planned use (adhesion) is not carried out, or after the use in a patient; as well as to assure the patient safety, since the use of the adhesive solely and exclusively for one patient is facilitated and made possible by using the single-dose prototype of suitable size for each application, thus the risk of cross-contamination between patients, a relatively high risk in those situations of unsuitable practice in which one and the same product is used to treat many patients, being reduced; additionally, they have easy handling , since the compositions of this invention can be produced in many presentation forms and sizes, in the form of solid film, all this depending on the surgery to be applied, reducing costs, facilitating the work of the surgeons, reducing times and conditioning the form of the resulting adhesive to the needs of each surgery. Furthermore, the adhesives provided by this invention facilitate wound apposition, thus providing a stable and regular force of biomechanical stress along the entire incisional route, thereby aiding in the maintenance of the tissue structure in the place of the lesion, especially, for example, in small tunnel type scleral or corneal incision in cataract surgery among other multiple applications.

Therefore, in one aspect, the invention relates to a composition in the form of film, comprising:
a) a layer (A) comprising a fibrinogen activator and a biocompatible material; and
b) a layer (B) comprising fibrinogen and a biocompatible material;
wherein the fibrinogen activator and the fibrinogen are not present simultaneously in one and the same layer.

In another aspect, the invention relates to a composition in the form of film comprising a compressed mixture of a lyophilisate comprising a fibrinogen activator and a lyophilisate comprising fibrinogen.

In another aspect, the invention relates to a composition in the form of film comprising a fibrinogen activator and fibrinogen, obtained by compressing of a mixture comprising (i) a lyophilisate comprising a fibrinogen activator and (ii) a lyophilisate comprising fibrinogen.

In another aspect, the invention relates to methods for obtaining the compositions in the form of dehydrated film indicated above.

In another aspect, the invention relates to a fibrin adhesive comprising one of said compositions in the form of film described above or obtainable by means of said methods.

In another aspect, the invention relates to said compositions or fibrin adhesive for use in inhibiting or stopping blood loss (hemorrhage) from a wound, or for use in adhering tissues. In a particular embodiment, said composition or fibrin adhesive is used for adhering damaged eye tissue, for example, eye tissue damaged in a traumatic or accidental manner by a lesion or as a result of an eye surgery or in any surgery which is thus required.

### Brief Description of the Drawings

Figure 1 is a photograph showing some prototypes of emulsions before the compression process. T-PLGA: corresponds to the solid formed by the dry thrombin and PLGA emulsion. F-PLGA: corresponds to the solid formed by the dry fibrinogen and PLGA emulsion.

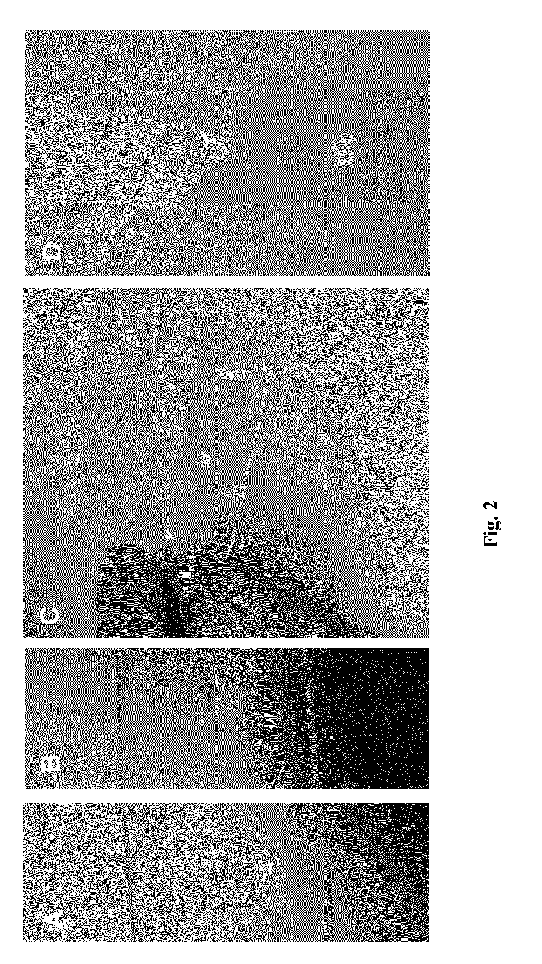

Figure 2 is a photograph showing the results of the in vitro adhesivity test of some of the different formulations of the active ingredients lyophilized, reconstituted and assayed with respect to their adhesive properties. A. Photograph of the hydrated lyophilisates and at initial time of mixing. B. Lyophilisates reconstituted 3 minutes after mixing. C. Thrombin and fibrinogen solutions after 5 minutes of mixing and incubation. D. Cover slips and slides adhered after 3 minutes of incubation at room temperature with the mixture of fibrin and thrombin after hydrating.
Figure 3 is an illustrative photograph of the *in vitro ex vivo* tissue adhesivity test of a composition provided by this invention. A. Film at initial time of incubation. B. Sealing test. C. Elasticity or malleability of the prototype assayed.

### Detailed Description of the Invention

### 1. Definitions

In the sense used herein, the term "fibrinogen activator" relates to a compound reacting with the fibrinogen to form fibrin. Non-limiting illustrative examples of fibrinogen activators include thrombin, prothrombin or a prothrombin complex concentrate (e.g., a mixture of factors II, VII, IX and X), spider venoms, snake venoms, for example batroxobin, reptilase (a mixture of batroxobin and factor XIIIa), bothrombin, calobin, fibrozyme, enzymes isolated from the *Bothrops jaracussus* venom, for example, BjussuSP-I, an enzyme similar to the thrombin isolated from the venom of the B. *jararacussus* snake, etc., and any mixture of said compounds [see, for example, Dascombe et al., Thromb. Haemost. 78:947-51 (1997); Hahn et al., J. Biochem. (Tokyo) 119:835-43 (1996); Fortova et al, J. Chromatogr. S. Biomed. Appl. 694:49-53 (1997); Andriao-Escarso et al., Toxicon. 35: 1043-52 (1997); and Sant'Ana et al., Biochimie. 90(3): p. 500-7 (2008)]. Preferably, the fibrinogen activator is a thrombin, more preferably, a mammal thrombin. As used herein, the term "thrombin" is defined below.

In the sense used herein, the term "fibrinogen", relates to a soluble blood plasma protein precursor of the fibrin and it includes any protein exhibiting the fibrinogen activity of the human fibrinogen. The fibrinogen activity of a protein can be determined by comparing the concentration of protein needed to form the same amount of fibrin clots to a solution of human fibrinogen at a concentration of 2.6 mg/mL, using the following assay: fibrin clots are formed in 96-well polystyrene microplates using fibrinogen (or the protein the fibrinogen activity of which is to be known) in TBS (Tris Buffer Saline) plus 1 nM human thrombin in TBS added to form the clot in a total volume of 200 µL. The clotting is evaluated by monitoring the change in the turbidity (A₄₀₅) for 1 hour at room temperature using a microplate reader. The fibrinogen can be isolated from the blood or plasma of a mammal by conventional methods, for example, those described in the international patent application published with the publication number WO 03/047530, etc., or it can be produced by means of the recombinant DNA technology such as described, for example, in Roy et al., J. Biol. Chem. 266:4758-4763 (1991), WO 03/047530, WO2010/004004, etc., or even from the fluids (blood, milk, etc.) of transgenic animals which contain a gene encoding fibrinogen (US 2006/174357, WO 95/22249, etc.), etc. Non-limiting illustrative examples of fibrinogen which can be used in the present invention include human fibrinogen, bovine fibrinogen, recombinant fibrinogen, etc.

In the sense used herein, the term "film", "sheet" or "lamina", relates to a three-dimensional, solid, substantially flat structure, the surface of which is generally greater than the thickness thereof. The thickness of said film can vary within a wide range, typically between approximately 1 nanometer (nm) and approximately 1 millimeter (mm), generally between approximately 0.1 mm and approximately 0.5 mm, commonly between approximately 0.2 mm and approximately 0.4 mm.

In the sense used herein, the term "wound", includes any disruption of continuity in the tissue of a subject. The tissue can be internal tissue, for example, an organ, etc., or external tissue, for example, the skin, etc.

The term "surgical wound" refers to all those solution of skin and/or mucosal and subjacent tissues continuity caused by the surgeon for the purpose of accessing any region of the organism.

In the sense used herein, the term "lyophilisate", relates to a product, composition or preparation obtained by means of a lyophilization process. The lyophilization also known as cryodehydration or drying by freezing is a method for separating water from a substance, substance being understood as pure products, mixtures, or preparations, including aqueous solutions which through freezing and subsequent subliming of the ice formed generally at reduced pressure, gives rise generally to a solid and dry material (lyophilisate) which can be reconstituted and hydrated with ease. A lyophilisate, as mentioned herein can be made up of only a single substance in lyophilisate form, or it can be formed by different substances, all of them in lyophilisate form.

As used herein, the term "biocompatible material", includes any pharmacologically inert natural or synthetic material which can form emulsions, and which can be implanted or incorporated in a living system or organism. In a particular embodiment, said biocompatible material is a polymer or copolymer which can form emulsions, such as for example, a polyanionic polysaccharide (e.g., hyaluronic acid, carboxymethylcellulose, chondroitin-6-sulfate, etc.), alginic acid and the derivatives thereof (e.g., alginates), polyvinyl alcohol (PVOH), poly(lactic acid) or PLA, poly(lactic-co-glycolic acid) (PLGA), polycaprolactone (PCL), etc., as well as copolymers thereof. In a particular embodiment, said biocompatible material is the PLGA, a biodegradable material approved by the North American Food and Drug Administration (FDA) for diverse medical applications and its fast biodegradability in the human body has been duly proven [Astete, C.E. and C.M. Sabliov, Synthesis and characterization of PLGA nanoparticles. J Biomater Sci Polym Ed, 2006. 17(3):247-89; Steinbüchel, A. and R.H. Marchessault, Biopolymers for medical and pharmaceutical applications. 2005, Germany. Willey-VCH Verlag. 160-177]. Advantageously, said biocompatible material is also biodegradable or resorbable, i.e., it can be hydrolyzed spontaneously and/or by the organism itself into the components thereof, which can be absorbed and/or eliminated such that they do not significantly interfere with the wound healing or with the tissue regeneration, and without causing any significant metabolic damage.

In the sense used herein, the term "subject" includes any mammal, including a human being.

In the sense used herein, the term "thrombin" relates to an enzyme with multiple functions in homeostasis, capable of catalyzing the rupture of the fibrinogen to form fibrin monomers, and includes any protein exhibiting the thrombin activity of the human thrombin. The thrombin activity of a protein can be determined by comparing the concentration of protein needed to form the same amount of fibrin clots to a solution of 1 nM human thrombin, using the following assay: fibrin clots are formed in 96-well polystyrene microplates using human fibrinogen at a concentration of 2.6 mg/mL in TBS (Tris Buffered Saline) plus thrombin (or the protein the thrombin activity of which is to be known) in TBS added to form the clot in a total volume of 200 µL. The clotting is evaluated by monitoring the change in the turbidity (A₄₀₅) for 1 hour at room temperature using a microplate reader. The thrombin can be isolated from the blood or plasma of a mammal by conventional methods, for example, those described by Miller & Copeland [Miller & Copeland, "Human thrombin: Isolation and stability", Experimental and Molecular Pathology, Volume 4, Issue 4, August 1965, Pages 431-437], Aizawa *et al*. [Aizawa P. et al., et al., "Large-scale preparation of thrombin from human plasma", Thrombosis Research, Volume 122, Issue 4, 2008, Pages 560-567], etc., or it can be produced by means of the recombinant DNA technology such as described, for example, in WO2007040162, WO2007/103447, WO2009/008821, etc., or even from the fluids (blood, milk , etc.) of transgenic animals which contain a gene encoding thrombin or prothrombin (for example, WO01/11952, etc.), etc. Non-limiting illustrative examples of thrombin which can be used in the present invention include the mammal thrombin, particularly, human thrombin or bovine thrombin, such as for example Thrombin-JMI^{®}, etc., recombinant thrombins, for example, the one marketed under the name Recothrom^{®}, or any protein, either of animal or human origin, transgenic or recombinant, exerting the fibrinogen activating function, being in this application in particular, preferably of human origin. The human thrombin is a glycoprotein enzyme formed by two polypeptide chains of 36 and 259 amino acids, respectively, bound by a disulphide bridge, which is formed from the precursor prothrombin in a reaction catalyzed by the thromboplastin enzyme in the presence of calcium ions (Ca²⁺).

Generally both the fibrinogen and the thrombin can be of natural origin (human or animal), transgenic or recombinant (modified animals, cell culture, etc.) providing said highly purified molecules for the application thereof in humans, having the hemostatic and bioadhesive activity typical of each of them in their human origin.

### 2. Composition of the invention

In one aspect, the invention relates to a composition in the form of film, hereinafter composition of the invention, selected from the group formed by:
[1] a composition in the form of film, hereinafter composition [1] of the invention, comprising:
   a) a layer (A) comprising a fibrinogen activator and a biocompatible material; and
   b) a layer (B) comprising fibrinogen and a biocompatible material;
   wherein the fibrinogen activator and the fibrinogen are not present simultaneously in one and the same layer; and
[2] a composition in the form of film, hereinafter composition [2] of the invention, comprising a compressed mixture of a lyophilisate comprising a fibrinogen activator and a lyophilisate comprising fibrinogen.

### 3. Composition [1] of the invention

### 3.1 Characterization of the composition [1] of the invention

In one aspect, the invention relates to a composition in the form of film, hereinafter composition [1] of the invention, comprising:
a) a layer (A) comprising a fibrinogen activator and a biocompatible material; and
b) a layer (B) comprising fibrinogen and a biocompatible material;
wherein the fibrinogen activator and the fibrinogen are not present simultaneously in one and the same layer.

### Layer A

The composition [1] of the invention comprises a layer (A) comprising a fibrinogen activator and a biocompatible and biodegradable material. The fibrinogen activator is preferably a thrombin, more preferably, a mammal thrombin, yet more preferably, human thrombin, for example Evithrom^{®} or bovine thrombin, such as for example Thrombin-JMI^{®}, or a recombinant thrombin, for example, the one marketed under the name Recothrom^{®}, which is a recombinant thrombin very similar to the human thrombin and generally any fibrinogen activator either of animal or human origin, of transgenic or recombinant origin being for this application in particular, preferably of human origin. In a particular embodiment, said fibrinogen activator is thrombin, particularly, human thrombin or a human recombinant thrombin. Advantageously, the fibrinogen activator has been subjected to a treatment to inactivate viruses, and/or has been produced by recombinant techniques. The amount of fibrinogen activator present in the layer (A) can vary within a wide range; in general, preferably, the amount of fibrinogen activator, for example thrombin, present in the layer (A), will depend on the amount of fibrinogen present in the layer (B). Thus, in a particular embodiment, the amount of fibrinogen activator present in the layer (A), expressed in International Units by centimeters squared (IU/cm²) is of at least 50.0 IU/cm², for example from 1.0 IU/cm² to 1,000,0 IU/cm², preferably from 50.0 IU/cm² to 500.0 IU/cm², more preferably from 75.0 IU/cm² to 250.0 IU/cm², yet more preferably, including 100.0, 125.0, 150.0, 175.0, 200.0, and 225.0 IU/cm².

The biocompatible material which can be present in the layer (A), can be any biocompatible material which can form emulsions, for example, a polyanionic polysaccharide, alginic acid and the derivatives thereof, PLA, PLGA, PCL, etc., preferably, PLGA. In a specific embodiment, said biocompatible material is PLGA with a monomer ratio of 50:50 (PLGA 50:50). The amount of biocompatible material present in the layer (A) can vary within a wide range depending, among others factors, on the nature of the biocompatible material; nevertheless, in a particular embodiment, the amount of biocompatible material present in the layer (A) is from at least 1.0 mg/cm² (milligram per centimeter squared), for example, from 2.0 mg/cm² to 60.0 mg/cm², preferably, from 4.0 to 55.0 mg/cm², including 5.0, 7.5, 10.0, 12.5, 15.0, 17.5, 20.0, 22.5, 25.0, 27.5, 30.0, 32.5, 35.0, 37.5, 40.0, 42.5, 45.0, 47.5, 50.0, and 52.5 mg/cm²; in a particular embodiment, the amount of biocompatible material present in the layer (A) is 12.5, 18.75, 25.0 or 30.0 mg/cm².

In a particular embodiment, said biocompatible material forms a matrix with a three-dimensional structure generating porosities both at surface level and at internal level wherein the fibrinogen activator is randomly distributed.

Said layer (A), in addition to a fibrinogen activator and a biocompatible material, can contain, if desired, a source of calcium ions (Ca²⁺), such as a calcium salt; although in principle, any pharmaceutically acceptable salt containing calcium ions could be used, in a particular and preferred embodiment, said source of calcium ions is calcium chloride (CaCl₂). The amount of calcium ions in the layer (A) can vary within a wide range; nevertheless, in a particular embodiment, the amount of calcium ions per centimeter squared is from at least 10 millimoles per centimeter squared (mmol/cm²), for example, from 10 to 100 mmol/cm², preferably from 15 to 85 mmol/cm², more preferably from 25 to 50 mmol/cm²; nevertheless, in a particular embodiment, the amount of calcium ions present in the layer (A) is 25, 35, or 45 mmol/cm².

Additionally, if desired, said layer (A) can contain a pharmaceutically acceptable excipient, for example, human albumin, sodium chloride, glycine, sterile water for injection, etc., or combinations thereof.

### Layer B

The composition [1] of the invention comprises a layer (B) comprising fibrinogen and a biocompatible material. Preferably, the fibrinogen is a mammal fibrinogen, more preferably, human fibrinogen. Advantageously, the fibrinogen has been subjected to a treatment to inactivate viruses. The amount of fibrinogen present in the layer (B) can vary within a wide range; nevertheless, in a particular embodiment, the amount of fibrinogen is from at least 20.0 mg of fibrinogen, for example, from 22.0 to 200.0 mg, preferably from 26.0 to 100.0 mg, more preferably from 30.0 to 60.0 mg.

The biocompatible material which can be present in the layer (B), can be any biocompatible material which can form emulsions, for example, a polymer or copolymer such as a polyanionic polysaccharide, alginic acid and the derivatives thereof, PLA, PLGA, PCL, etc., or any copolymer of the above, preferably, PLGA. In a specific embodiment, said biocompatible material is PLGA with a monomer ratio of 50:50 (PLGA 50:50). The amount of biocompatible material present in the layer (B) can vary within a wide range depending, among other factors, on the nature of the biocompatible material; nevertheless, in a particular embodiment, the amount of biocompatible material present in the layer (B) is from at least 1.0 mg/cm² (milligram per centimeter squared), for example, from 2.0 mg/cm² to 60.0 mg/cm², preferably from 4.0 to 55.0 mg/cm², including 5.0, 7.5, 10.0, 12.5, 15.0, 17.5, 20.0, 22.5, 25.0, 27.5, 30.0, 32.5, 35.0, 37.5, 40.0, 42.5, 45.0, 47.5, 50.0, and 52.5 mg/cm²; in a particular embodiment, the amount of biocompatible material present in the layer (A) is 12.5, 18.75, 25.0 or 30.0 mg/cm².

The biocompatible material present in the layer (B) can be the same as or different from the biocompatible material present in the layer (A); thus in a particular embodiment, the biocompatible material present in the layer (B) is the same as the biocompatible material present in the layer (A), whereas in another particular embodiment, the biocompatible material present in the layer (B) is different from the biocompatible material present in the layer (A). In a specific embodiment, the biocompatible material present in the layer (B) is the same as the biocompatible material present in the layer (A) and said biocompatible material is PLGA.

Said layer (B), in addition to fibrinogen and a biocompatible material, can contain, if desired, a cross-linking agent stabilizing the clot by means of cross-linking (reticulation) the alpha (α) and gamma (γ) chains of the fibrin coming from the hydrolysis of the fibrinogen. In a particular embodiment, said cross-linking agent is the clotting factor XIII (factor XIII); nevertheless, other cross-linking agents used in formulating fibrin adhesives known by the person skilled in the art could be used. When the factor XIII is used as cross-linking agent, the latter present in the layer (B) in an amount expressed in International Units per centimeter squared (IU/cm²) of at least 1 IU/cm², for example, from 3.0 to 60.0 IU/cm², preferably from 10.0 to 50.0 IU/cm², including 15.0, 20.0, 30.0, and 45.0 IU/cm². Preferably, if the factor XIII is present in the layer (B), the factor XIII is mammal factor XIII, preferably human or bovine factor XIII, more preferably, human factor XIII. Advantageously, said factor XIII has been subjected to a treatment to inactivate viruses, and/or has been produced by recombinant techniques.

Optionally, said layer (B) can contain, if desired, a fibrinolysis inhibitor such as an anti-fibrinolytic agent, for example, aprotinin, tranexamic acid, etc., preferably aprotinin.

When aprotinin (bovine version of the basic pancreatic trypsin inhibitor or BPTI) is used as anti-fibrinolytic agent, that compound is present in the layer (B) in an amount expressed in Kallidinogenase Inactivator Unit per centimeter squared (KIU/cm²), of at least 100.0 KIU/cm², for example, from 100.0 to 3,300.0 KIU/cm², more preferably from 150.0 to 1,600.0 KIU/cm², including 250.0-850.0 KIU/cm². Advantageously, said anti-fibrinolytic agent has been subjected to a treatment to inactivate viruses. Nevertheless, in a particular and preferred embodiment, the layer (B) does not contain any fibrinolysis inhibitor since the hypersensitivity reactions which they cause, mainly when they are applied at systemic level, as well as the toxicity of the tranexamic acid at neurosurgical level, are known.

Likewise, if desired, said layer (B) can contain plasminogen, the main component of the fibrinolytic system of the organism, i.e., it acts in dissolving blood clots. When the plasminogen is present in the layer (B), said compound can be present in an amount per mg of fibrinogen of at least 0.01 milligrams (mg), for example, from 0.01 to 1.00 mg, preferably from 0.10 to 0.80 mg, more preferably from 0.12 to 0.50 mg. Advantageously, said plasminogen has been subjected to a treatment to inactivate viruses. Nevertheless, in a particular and preferred embodiment, the layer (B) does not contain plasminogen.

Additionally, if desired, said layer (B) can contain a pharmaceutically acceptable excipient, for example, human albumin, glycine, sodium chloride, sodium citrate, polysorbate 80, creatinine, sterilized water for injections, etc., or combinations thereof.

The composition [1] of the invention comprises a layer (A) and a layer (B); both layers are solid and are in adjacent positions, or one on top of the other. In a particular embodiment, the layer (B) is on top of the layer (A). In another particular embodiment, the layer (A) is on top of the layer (B).

The composition [1] of the invention can contain two or more layers; in any case, the condition of which the fibrinogen activator and the fibrinogen are not present simultaneously in the same layer must be maintained for the purpose of preventing or minimizing the risk that the chemical reaction between the compounds is triggered unintentionally. In a particular embodiment, the composition [1] of the invention is bilayer, i.e., only comprises, or is made up of, 2 layers, in which case, one of them is the layer (A) and the other, the layer (B). In another particular embodiment, the composition [1] of the invention is multilayer, i.e., comprises three or more layers, in which case, at least one of them is the layer (A) and another is the layer (B); in this embodiment of the invention, the composition [1] of the invention can contain, for example, 2 layers (A) and 1 layer (B) which can be arranged in any arrangement, for example, forming a structure of layers of type (from bottom to top) (A)-(B)-(A), etc., or, alternatively, 1 layer (A) and 2 layers (B), arranged in any arrangement, for example, forming a structure of layers of type (from bottom to top) (B)-(A)-(B), etc. In any case, the condition of which the fibrinogen activator and the fibrinogen are not present simultaneously in one and the same layer must be maintained. In a particular embodiment specifically for applications in the field of ophthalmology, the composition [1] of the invention is bilayer due to, among other reasons, the limitations associated with the thickness of the film for that type of applications; nevertheless, for other applications which do not have that limiting factor, the composition [1] of the invention is a bilayer or multilayer composition.

The thickness of the film of the composition [1] of the invention can vary within a wide range; nevertheless, in a particular embodiment, the thickness of the film is of at least 1.0 nm, generally between approximately 1.0 nm and approximately 1.0 mm, preferably between approximately 0.1 mm and approximately 0.5 mm, more preferably between approximately 0.2 mm and approximately 0.4 mm.

An additional characteristic of the composition [1] of the invention consists of the fact that it is a "pre-manufactured" composition, i.e., it is prepared outside the site of treatment and is adapted for storage and transportation. The fact that the composition [1] of the invention is in the form of film enables making it in the desired form for application and, furthermore, it allows using only the amount of product needed either by manufacturing the films in different surface area measurements and/or adjusting it with respect to the shape of the wound according to the surgical application.

The composition [1] of the invention (a solid film) in contact with an aqueous medium can exert its adhesive activity since in said aqueous medium the layers (A) and (B) of the composition [1] of the invention are hydrated, which allow destabilizing the emulsion and the subsequent interaction of the fibrinogen activator and the fibrinogen, such that when the fibrinogen activator is contacted with the fibrinogen, the chemical reaction entailing the formation of fibrin from the fibrinogen is triggered. The fibrin forms a kind of net around the wound mimicking the final step of the clotting cascade needed for wound healing. Non-limiting illustrative examples of said aqueous medium include body fluids, for example, blood, serum , vitreous humor, etc., as well as water-based non-bodily fluids, for example, water, saline, Ringer's solution, calcium chloride, etc.

### 3.2 Method for obtaining the composition [1] of the invention

The composition [1] of the invention can be obtained by means of a method comprising, on one hand, a layer A formed by an emulsion comprising a fibrinogen activator and a biocompatible material, which after a process of drying is subjected to pressing to form a sheet. On the other hand, the layer B is formed by an emulsion comprising fibrinogen and a biocompatible material, which after a process of drying is subjected to pressing to form a second sheet separately. Finally a compression is performed where the sheet of the layer A is deposited on the sheet of the layer B to give rise to the composition [1] of the invention in the form of bilayer or bilaminar film.

Therefore, in another aspect, the invention relates to a method for obtaining a composition in the form of film, wherein said composition in the form of film comprises: a) a layer (A) comprising a fibrinogen activator and a biocompatible material; and b) a layer (B) comprising fibrinogen and a biocompatible material; wherein the fibrinogen activator and the fibrinogen are not present simultaneously in one and the same layer, said method comprising the steps of:
i) depositing an emulsion comprising a fibrinogen activator and a biocompatible material on a surface comprising an anti-adherent material, drying and pressing the solid generated until forming a sheet [layer (A)];
ii) depositing an emulsion comprising fibrinogen and a biocompatible material on a surface comprising an anti-adherent material, drying and pressing the solid generated until forming a sheet [layer (B)];
iii) depositing the sheet comprising fibrinogen and a biocompatible material [layer (B)] on the sheet comprising a fibrinogen activator and a biocompatible material [layer (A)]; or, alternatively, depositing the sheet comprising a fibrinogen activator and a biocompatible material [layer (A)] on the sheet comprising fibrinogen and a biocompatible material [layer (B)]; and
iv) subjecting the product of step iii) to a process of compression until compacting the two layers and obtaining said composition in the form of film comprising said layers (A) and (B) and in which the fibrinogen activator and the fibrinogen are not present simultaneously in one and the same layer.

The emulsion comprising the fibrinogen activator and a biocompatible material can be obtained by conventional methods, mixing said fibrinogen activator in aqueous solution with said biocompatible material in the presence of an organic solvent in which said biocompatible material is soluble on a solid surface comprising an anti-adherent material, and subjecting said aqueous and organic solutions to stirring until forming the emulsion. In a particular embodiment, said solid surface comprising an anti-adherent material is a solid surface made or configured in any shape or configuration desired, for example, a flat surface, a rippled surface, etc., it is inside a mold, inside a tube, etc., having the dimensions desired, and it is completely or substantially formed by an anti-adherent material or is completely or substantially coated with an anti-adherent material; although virtually any anti-adherent material can be used, in a particular embodiment, said anti-adherent material is Teflon [polytetrafluoroethylene (PFTE)] or the like. In a particular embodiment, said fibrinogen activator is in the lyophilisate form and is reconstituted in an aqueous solution preferably, the biocompatible material is PLGA, the organic solvent is chloroform, dichloromethane or, generally, any organic solvent, and the stirring is carried out by means of ultrasound under suitable conditions, for example, by means of the application of a power of at least 20 watts (w), for example between 25 and 150 w, preferably between 30 and 120 w, including 30, 50, 70 and 100 w, for a time of at least 0.1 seconds (s), for example between 10.0 and 120.0 s, preferably between 30.0 and 100.0 s, including 40.0, 60.0 and 80.0 s. Said method allows generating emulsions. Then, the resulting emulsion is subjected to a process of drying by conventional methods, for example, leaving to dry at room temperature, drying in oven, driers and/or lyophilisate. In a particular embodiment, said emulsion comprising a fibrinogen activator and a biocompatible material deposited on a solid surface comprising an anti-adherent material, is subjected to a process of drying at a temperature comprised between approximately 18°C and approximately 25°C, typically about 20°C, for a time period comprised between 8 and 16 hours, under hood until drying and solidifying the emulsion, a solid layer being generated. Said solid layer comprising a fibrinogen activator and a biocompatible material, for example, PLGA, etc., has a water content equal to or less than 20% by weight of the total weight of said layer, generally equal to or less than 10%, typically equal to or less than 5%, preferably equal to or less than 3%, yet more preferably equal to or less than 1.0% by weight with respect to the total weight of said solid layer comprising a fibrinogen activator and a biocompatible material, therefore said solid layer comprising a fibrinogen activator and a biocompatible material is substantially water-free, i.e., it is substantially dehydrated or dry. The water content of a layer (or film) such as the one mentioned above can be determined by conventional methods, for example, assessing the water loss by weight or analytical techniques such as Karl-Fisher titration or infrared spectroscopy, among others. Subsequently, said dry solid layer comprising a fibrinogen activator and a biocompatible material is subjected to a process of pressing or compression by conventional methods, for example, by means of direct compression, whereby a solid, dry and pressed sheet or layer comprising a fibrinogen activator, for example, thrombin, and a biocompatible material [layer (A)] is obtained.

Likewise, the emulsion comprising fibrinogen and a biocompatible material can be obtained by conventional methods, mixing said fibrinogen in aqueous solution with said biocompatible material in the presence of an organic solvent in which said biocompatible material is soluble and subjecting said aqueous and organic solutions to stirring until the formation of the emulsion on a solid surface comprising an anti-adherent material. In a particular embodiment, said solid surface comprising an anti-adherent material is a solid surface made or configured in any shape desired, for example, a flat surface, a rippled surface, etc., in the form of a mold, in a tube, etc., having the dimensions desired, and is completely or substantially formed by an anti-adherent material or completely or substantially coated with an anti-adherent material, for example, Teflon or the like. In a particular embodiment, said fibrinogen is in the form of lyophilisate and is reconstituted in an aqueous solution preferably, the biocompatible material is PLGA, the organic solvent is chloroform, dichloromethane or, generally, any organic solvent, and the stirring is carried out by means of ultrasound under suitable conditions, for example, by means of the application of a power of at least 20 watts (w), for example between 25 and 150 w, preferably between 30 and 120 w, including 30, 50, 70 and 100 w, for a time of at least 0.1 second (s), for example, between 10.0 and 120.0 s, preferably between 30.0 and 100.0 s, including 40.0, 60.0 and 80.0 s. Said method allows generating emulsions. Then, the resulting emulsion is subjected to a process of drying by conventional methods, for example, leaving to dry at room temperature, drying in oven, driers and/or lyophilisate. In a particular embodiment, said emulsion comprising fibrinogen and a biocompatible material deposited on said solid surface comprising an anti-adherent material, is subjected to a process of drying at a temperature comprised between approximately 18°C and approximately 25°C, typically about 20°C for a time period comprised between 8 and 16 hours, under hood until drying and solidifying the emulsion, a solid layer being generated. Said solid layer comprising fibrinogen and a biocompatible material, for example, PLGA, etc., has a water content equal to or less than 20% by weight of the total weight of said layer, generally equal to or less than 10%, typically equal to or less than 5%, preferably equal to or less than 3%, yet more preferably equal to or less than 1.0% by weight with respect to the total weight of said solid layer comprising fibrinogen and a biocompatible material, therefore said solid layer comprising fibrinogen and a biocompatible material is substantially water-free, i.e., it is substantially dehydrated or dry. The water content of a layer (or film) such as the one mentioned above can be determined by conventional methods, for example, assessing the water loss by weight or analytic techniques such as Karl-Fisher titration or infrared spectroscopy, among others. Subsequently, said dry solid layer comprising fibrinogen and a biocompatible material, is subjected to a process of pressing or compression by conventional methods, for example, by means of direct compression, whereby a solid, dry and pressed sheet or layer comprising fibrinogen and a biocompatible material [layer (B)] is obtained.

The person skilled in the art will understand that steps i) and ii) identified above can be performed in any order, i.e., performing, for example, first step i) and then step ii), or vice versa; all those alternatives being included within the scope of the present invention.

Then, once the layers (A) and (B) are obtained, the sheet comprising fibrinogen and a biocompatible material [layer (B)] is deposited on the sheet comprising a fibrinogen activator and a biocompatible material [layer (A)], or, alternatively, the sheet comprising a fibrinogen activator and a biocompatible material [layer (A)] is deposited on the sheet comprising fibrinogen and a biocompatible material [layer (B)] [step iii)]. In the event that said layers (A) and/or (B) did not have the suitable consistency and strength to enable handling them independently they can be subjected to a process of compression or pressing to generate a sheet or film with the suitable consistency and strength. In a particular embodiment, the layer (B) is separated from the solid surface comprising an anti-adherent material and is deposited on the layer (A) which is maintained on the solid surface comprising an anti-adherent material on which it has been formed. In another particular embodiment, the layer (A) is separated from the solid surface comprising an anti-adherent material and is deposited on the layer (B) which is maintained on the solid surface comprising an anti-adherent material on which it has been formed. Alternatively, the layer (B) is separated from the solid surface comprising an anti-adherent material and is deposited on the layer (A) which, has in turn been separated from the solid surface comprising an anti-adherent material on which it has been formed, and has been deposited on another solid surface comprising an anti-adherent material, or, the layer (A) is separated from the solid surface comprising an anti-adherent material and is deposited on the layer (B) which, has in turn been separated from the solid surface comprising an anti-adherent material on which it has been formed, and has been deposited on another solid surface comprising an anti-adherent material. The person skilled in the art will understand that this step of the method can be performed in many ways, all of them included within the scope of the present invention.

Subsequently, the product resulting from step iii) of the method described above is subjected to a process of compression until compacting the two (A) and (B) layers. Said process of compression can be any process of compression or conventional pressing suitable for compressing materials into the form of layers, laminas or sheets, for example, by means of direct compression until obtaining a bilayer film comprising the layers A and B defined previously, and in which the fibrinogen activator and the fibrinogen are not present simultaneously in one and the same layer.

In a particular embodiment, a method is provided for obtaining a composition in the form of bilayer film, wherein said composition in the form of film comprises: a) a layer (A) comprising a fibrinogen activator and a biocompatible material; and b) a layer (B) comprising fibrinogen and a biocompatible material; wherein the fibrinogen activator and the fibrinogen are not present simultaneously in one and the same layer, comprising the steps of:
a) depositing an emulsion comprising a fibrinogen activator and a biocompatible material on a solid surface comprising an anti-adherent material,
b) leaving said emulsion deposited in the step a) to dry until forming a dehydrated solid comprising a fibrinogen activator and a biocompatible material;
c) pressing the dehydrated solid generated in step b) until forming a sheet [layer (A)];
d) depositing an emulsion comprising fibrinogen and a biocompatible material on a solid surface comprising an anti-adherent material;
e) leaving said emulsion deposited in step d) to dry until forming a dehydrated solid comprising fibrinogen and a biocompatible material;
f) pressing the dehydrated solid generated in step e) until forming a sheet [layer (B)];
g) depositing the sheet comprising fibrinogen and a biocompatible material [layer (B)] on the sheet comprising a fibrinogen activator and a biocompatible material [layer (A)] or alternatively depositing the sheet comprising fibrinogen and a biocompatible material [layer (A)] on the sheet comprising a fibrinogen activator and a biocompatible material [layer (B)]; and
h) subjecting the product resulting from step g) to a process of compression until compacting the two layers and obtaining said composition in the form of bilayer film comprising said layers (A) and (B) and in which the fibrinogen activator and the fibrinogen are not present simultaneously in one and the same layer.

In a particular embodiment, said solid surface comprising an anti-adherent material is a solid surface made or configured in any shape desired, for example, a flat surface, a rippled surface, etc., in the form of tube, mold, etc., having the dimensions desired, and is completely or substantially formed by an anti-adherent material or completely or substantially coated with an anti-adherent material; although virtually any anti-adherent material can be used, in a particular embodiment, said anti-adherent material is Teflon or the like.

The way to carry out the steps a)-h) has already been described above in relation with the general method [steps i)-iv)] and are incorporated herein by reference.

In a particular embodiment, the fibrinogen activator and the fibrinogen used in the preparation of said emulsions are in the form of lyophilisate, therefore they must be reconstituted with an aqueous medium, for example, water, preferably sterile water, before mixing it with the biocompatible material. Obtaining said compounds in the form of lyophilisate is described below and is exemplified in Example 1.

In a particular embodiment, this method is carried out in a mold in which the surface of the mold contacts with the emulsions and wherein the layers (A) and (B) are generated in which both layers are compressed to generate the bilayer film, is of an anti-adherent material or, alternatively, it is completely or substantially coated with an anti-adherent material. In a particular embodiment, said anti-adherent material is Teflon or the like. Therefore, in a particular embodiment, the method for drying the emulsions is carried out in a suitable mold in which said mold contains a Teflon coat whereas the method of compression is performed in a press coated by aluminium foil which has anti-adherent properties with respect to the dry solid forming the layers (A) and (B) for the individual or bilayer compression thereof.

An additional characteristic of the composition [1] of the invention consists of the fact that it is a composition the structure, size and "pre-manufactured" final shape of which allows the direct application thereof, i.e., it is prepared outside the site of treatment and is adapted for storage and transportation. The fact that the composition [1] of the invention is in the form of film enables adjusting it in the desired form for application; furthermore, it can be presented in the form of single-dose or customized doses, such that only the amount of product needed is used since by selecting the prototype (product or formulation giving rise to the fibrin adhesive) of suitable size for each application the use of the adhesive needed exclusively to each patient is facilitated and made possible.

### 4. Composition [2] of the invention

In another aspect, the invention relates to a composition in the form of film, hereinafter composition [2] of the invention, comprising a compressed mixture of a lyophilisate comprising a fibrinogen activator and a lyophilisate comprising fibrinogen.

In a particular embodiment, said composition [2] of the invention is a composition in the form of film comprising a fibrinogen activator and fibrinogen obtained by compressing a mixture comprising (i) a lyophilisate comprising a fibrinogen activator and (ii) a lyophilisate comprising fibrinogen.

The composition [2] of the invention can be obtained by means of a method comprising the compression of a mixture comprising (i) a lyophilisate comprising a fibrinogen activator and (ii) a lyophilisate comprising fibrinogen.

A lyophilisate comprising a fibrinogen activator is a lyophilisate which is made up only of a fibrinogen activator in the form of lyophilisate, or which contains a fibrinogen activator and other substances, all of them in the form of lyophilisate. Preferably, the fibrinogen activator is a thrombin, such as a mammal thrombin, more preferably human or bovine thrombin, yet more preferably human thrombin. Advantageously, the fibrinogen activator has been subjected to a treatment to inactivate viruses, and/or has been produced by recombinant techniques, before the lyophilization thereof. The amount of fibrinogen activator present in the composition [2] of the invention can vary within a wide range; generally, the amount of fibrinogen activator will depend on the amount of fibrinogen. Thus, in a particular embodiment, the amount of fibrinogen activator present in the composition [2] of the invention expressed in International Units per centimeter squared (IU/cm²) is of at least 100.0 IU/cm², for example from 100.0 IU/cm² to 2,500.0 IU/cm², preferably from 150.0 IU/cm² to 1,500.0 IU/cm², more preferably from 250.0 IU/cm² to 1,000.0 IU/cm².

In a particular embodiment, the lyophilisate comprising a fibrinogen activator is a lyophilisate which is made up only of a fibrinogen activator in the form of lyophilisate. In another particular embodiment, said lyophilisate comprising a fibrinogen activator is a lyophilisate containing a fibrinogen activator and other substances, all of them in the form of lyophilisate. Said substances may include a source of calcium ions (Ca²⁺) as well as one or more pharmaceutically acceptable excipients, for example, human albumin, sodium chloride, glycine, etc., or combinations thereof. Although, in principle, any pharmaceutically acceptable salt containing calcium ions could be used, in a particular and preferred embodiment, said source of calcium ions is calcium chloride (CaCl₂). The amount of calcium ions present in the composition [2] of the invention can vary within a wide range; nevertheless, in a particular embodiment, the amount of calcium ions per centimeter squared is of at least 10.0 millimoles per centimeter squared (mmol/cm²), for example from 10.0 to 100.0 mmol/cm², preferably from 15.0 to 85.0 mmol/cm², more preferably from 25.0 to 50.0 mmol/cm²; nevertheless, in a particular embodiment, the amount of calcium ions present in the layer (A) is 25.0, 35.0 or 45.0 mmol/cm².

The lyophilisate comprising a fibrinogen activator can be obtained by conventional methods, for example, by means of a method which comprises subjecting an aqueous solution comprising a fibrinogen activator to one or more freezing-subliming cycles for the purpose of removing water from the solid to the gaseous state without passing through the liquid state. To accelerate the process several freezing-subliming cycles can be used. In a particular embodiment, a solution comprising a fibrinogen activator is frozen by any conventional freezing method at a temperature equal to or less than -20°C and down to -196°C, including, for example, -80°C in a specific embodiment, and, is then subjected to subliming under suitable conditions, advantageously at reduced pressure; although the pressure can vary within a wide range, nevertheless, in a particular embodiment, the pressure is equal to or less than 0.800 millibars (mbars) [80 Pa], for example between 0.001 and 0.750 mbars [0.1 and 75 Pa], typically between 0.010 and 0.500 mbars [1.0 and 50.0 Pa] of the frozen aqueous solution comprising the fibrinogen activator. Said process can be performed by generating a change of temperature from the freezing temperature of the sample (approximately -80°C) to an end temperature of the sample of approximately 10°C at the end of the process. The change of temperature is performed depending on the time of lyophilisate to which the sample is subjected to, for example, between 1 and 24 hours, normally between 2 and 12 hours, preferably between 3 and 8 hours, including 4, 5, 6 and 7 hours. By operating in the manner indicated above, a lyophilisate comprising a fibrinogen activator such as thrombin which can be rehydrated (reconstitution) with ease, which preserves the physicochemical and biological properties of the thrombin after the reconstitution thereof [Examples 1 and 2], is obtained. The process of lyophilization is carried out, generally, in a freeze-drier.

A lyophilisate comprising fibrinogen is a lyophilisate which is made up only of fibrinogen and/or excipients in the form of lyophilisate, or which contains fibrinogen and other substances, all of them in the form of lyophilisate. Preferably, the fibrinogen is a mammal fibrinogen, more preferably human fibrinogen. Advantageously, the fibrinogen has been subjected to a treatment to inactivate viruses before the lyophilization thereof. The amount of fibrinogen present in the composition [2] of the invention can vary within a wide range; nevertheless, in a particular embodiment, the amount of fibrinogen is of at least 50.0 mg of fibrinogen, for example from 100.0 to 600.0 mg, preferably from 75.0 to 300.0 mg, more preferably from 50.0 to 180.0 mg.

In a particular embodiment, the lyophilisate comprising fibrinogen is a lyophilisate which is made up only of fibrinogen in the form of lyophilisate. In another particular embodiment, said lyophilisate comprising fibrinogen is a lyophilisate containing fibrinogen and other substances, all of them in the form of lyophilisate. Said substances can include a cross-linking agent, for example, factor XIII; a anti-fibrinolytic agent, for example, aprotinin, tranexamic acid, etc.; plasminogen; and one or more pharmaceutically acceptable excipients, for example, human albumin, glycine, sodium chloride, sodium citrate, polysorbate 80, creatinine, etc., or combinations thereof.

If the composition [2] of the invention contains a cross-linking agent, for example, factor XIII, said cross-linking agent (factor XIII) can be present in an amount expressed in IU/cm², of at least 8.0 IU/cm², for example, from 17.0 to 102.0 IU/cm², preferably from 12.0 to 51.0 IU/cm², yet more preferably between 8.0 and 30.0 IU/cm². Preferably, said factor XIII is mammal factor XIII, preferably human or bovine factor XIII, more preferably, human factor XIII. Advantageously, said factor XIII has been subjected to a treatment to inactivate viruses, and/or has been produced by recombinant techniques.

Likewise, if the composition [2] of the invention contains an anti-fibrinolytic agent, for example, aprotinin, tranexamic acid, etc., preferably aprotinin, said anti-fibrinolytic agent (aprotinin) can be present in an amount expressed in KIU/cm² of at least 1,600.0 KIU/cm², for example from 3,000.0 to 20,000.0 KIU/cm², more preferably from 2,500.0 to 10,000.0 KIU/cm², including 1,600.0 to 6,000.0 KIU/cm². Advantageously, said anti-fibrinolytic agent has been subjected to a treatment to inactivate viruses. Nevertheless, in a particular and preferred embodiment, the composition [2] of the invention does not contain any fibrinolysis inhibitor.

Similarly, if the composition [2] of the invention contains plasminogen, said compound can be present in an amount per mg of fibrinogen of at least 0.040 mg, for example from 0.080 to 0.500 mg, preferably from 0.061 to 0.300 mg, more preferably from 0.040 to 0.20 mg. Advantageously, said plasminogen has been subjected to a treatment to inactivate viruses. Nevertheless, in a particular and preferred embodiment, the composition [2] of the invention does not contain plasminogen.

The lyophilisate comprising fibrinogen can be obtained by conventional methods, for example, by means of a method which comprises subjecting an aqueous solution comprising fibrinogen to one or more freezing-subliming cycles. To accelerate the process several freezing-subliming cycles can be used. In a particular embodiment, a solution comprising fibrinogen is frozen by any conventional freezing method at a temperature equal to or less than -20°C and down to -196°C, including, for example, -80°C in a specific embodiment, and, it is then subjected to subliming under suitable conditions, advantageously at reduced pressure; although the pressure can vary within a wide range, nevertheless, in a particular embodiment, the pressure is equal to or less than 0.800 mbars [80,0 Pa], for example between 0.001 and 0.750 mbars [0.1 and 75 Pa], typically between 0.010 and 0.500 mbars [1.0 and 50.0 Pa] of the frozen aqueous solution comprising fibrinogen. Said process can be performed by generating a change of temperature from the freezing temperature of the sample (approximately -80°C) to an end temperature of the sample of approximately 4°C at the end of the process of freezing-subliming. The change of temperature is performed depending on the time of lyophilisate to which the sample is subjected to, for example, between 1 and 24 hours, normally between 2 and 12 hours, preferably between 3 and 8 hours, including 4, 5, 6 and 7 hours. By operating in the manner indicated above, a lyophilisate comprising fibrinogen which can be rehydrated (reconstitution)with ease, which preserves the physicochemical and biological properties of the fibrinogen after the reconstitution thereof [Examples 1 and 2], is obtained. The process of lyophilization is carried out, generally, in a freeze-drier.

To obtain the composition [2] of the invention a mixture of said (i) lyophilisates comprising a fibrinogen activator and (ii) lyophilisate comprising fibrinogen are made; said mixture in the suitable amounts of the lyophilisates of the two components of the composition [2] is made for the purpose of assuring a homogenous distribution thereof, and they are subsequently subjected to a process of compression under suitable conditions which allow obtaining a film from said lyophilisates. The pressure to be applied can vary within a wide range, nevertheless, in a particular embodiment, said mixture of lyophilisates is subjected to a pressure comprised between approximately 150.0 bars (150 x 10⁵ Pa) and approximately 240.0 bars (240 x 10⁵ Pa), at a temperature comprised between approximately 20°C and approximately 200°C, typically comprised between approximately 30°C and approximately 60°C, advantageously lower than 60°C, due to the fact that at that temperature the fibrinogen is denatured, preferably at approximately 50°C for a variable time period, typically comprised between 1 and 10 minutes, preferably between 3 and 7 minutes, particularly about 5 minutes. This process of compression can be carried out in conventional pressing equipment, for example, in a hydraulic press. Advantageously, the two metal plates of the hydraulic press are covered with a suitable anti-adherent material, for example, aluminium foil, Teflon, etc., before placing the mixture of the lyophilisates for the purpose of preventing or minimizing the adhesion thereof to the metal plates of the hydraulic press. By operating in the manner indicated above, a solid film comprising a compressed mixture of a lyophilisate, which in turn comprises a fibrinogen activator and a lyophilisate comprising fibrinogen is obtained, making up an additional aspect of the invention. The thickness of the film of the composition [2] of the invention can vary within a wide range; nevertheless, in a particular embodiment, the thickness of the film is of at least 1.00 nm, generally between approximately 1.00 nm and approximately 1.00 mm, preferably between approximately 0.05 mm and approximately 0.50 mm, more preferably between approximately 0.10 mm and approximately 0.30 mm.

An additional characteristic of the composition [2] of the invention consists of the fact that it is a composition the structure, size and final "pre-manufactured" shape of which allows the direct application thereof, i.e., it is prepared outside the site of treatment and is adapted for storage and transportation. The fact that the composition [2] of the invention is in the form of film enables adjusting it in the desired form for application; furthermore, it can be presented in the form of single-dose or customized doses, such that only the amount of product needed is used since by selecting the prototype (product or formulation giving rise to the fibrin adhesive) of suitable size for each application the use of the adhesive needed exclusively to each patient is facilitated and made possible.

To promote the adhesive activity of the composition [2] of the invention (a solid film) this is required to contact with an aqueous medium, since in said aqueous medium the lyophilisates are hydrated and the reaction of the fibrinogen activator and the fibrinogen is achieved, allowing close contact between said components triggering the chemical reaction for the production of fibrin and the reticulation thereof from said components. The fibrin forms a kind of net around the wound imitating the last step of the clotting cascade needed for wound healing. Non-limiting illustrative examples of said aqueous medium include body fluids, for example, blood, serum, vitreous humor, etc., as well as water-based non-bodily fluids, for example, water, saline or saline solution, Ringer's solution, calcium chloride solution, etc.

Both the composition [1] of the invention and the composition [2] of the invention can include in their formulation any other type of molecule with pharmacological activity; non-limiting illustrative examples of said molecules with pharmacological activity which can be present in said compositions [1] and/or [2] of the invention include antibiotics, anti-fungal agents, anti-inflammatory agents, antiseptics, antivirals, cytokines, cytostatic agents, growth factors, proteins, peptides, procoagulants or even gene therapy agents which can be included in the formulations by itself or combined with one another which do not affect the adhesive and hemostatic function of the compositions [1] and/or [2] of the invention and which exert a therapeutic action on the patient or organism in which it will be used.

### 5. Applications of the Composition of the Invention

The composition of the invention, both the composition [1] of the invention and the composition [2] of the invention, can be used in the preparation of fibrin adhesives; to that end, said composition of the invention needs to be hydrated.

Therefore, in a particular embodiment, the invention relates to a fibrin adhesive comprising a hydrated composition in the form of film selected from the composition [1] of the invention and the composition [2] of the invention. Said fibrin adhesive can be activated by means of hydrating said composition of the invention with an aqueous medium; preferably, said aqueous medium is a medium suitable for application on the human or animal body; non-limiting illustrative examples of said aqueous medium suitable for application on the human or animal body include saline solution or saline, aqueous calcium chloride solution, Ringer's solution, sterilized water, for example, sterile water for injection, etc. The composition of the invention can be hydrated by conventional methods, for example, by contacting the composition [1] of the invention or the composition [2] of the invention with a suitable liquid, such as an aqueous medium, right before its application, generally, a few minutes before using it, for example, submerging the composition of the invention in an aqueous medium, or applying said aqueous medium directly on the composition of the invention by means of conventional techniques, for example, by means of spraying or the like.

In the sense used herein, the term "hydrated", applied to the composition of the invention means that said composition has been contacted with an aqueous medium and has a water content greater than 30% by weight of the total weight of the composition, generally equal to or greater than 40%, typically equal to or greater than 50%, commonly equal to or greater than 60%, advantageously equal to or greater than 70%, preferably equal to or greater than 80%, more preferably equal to or greater than 85%, still more preferably equal to or greater than 95% by weight with respect to the total weight of said hydrated composition of the invention. The water content of the hydrated composition of the invention resulting from contacting the composition of the invention with an aqueous medium can be determined by conventional methods, for example, by means of drying in an oven or in a vacuum stove, dried by infrared radiation, etc., and the corresponding assessment of the weight loss.

Therefore, the composition of the invention, both the composition [1] of the invention and the composition [2] of the invention, as well as the fibrin adhesive provided by this invention can be used in the applications typical of the fibrin adhesives, for example, in (i) hemostasis, wherein the fibrin clot acts as a hemostatic barrier and reduces the risk of infiltrates and/or hemorrhages, (ii) tissue adhesion (of animals), due to its adhesive properties the fibrin is connected in a non-traumatic manner with the tissues forming a strong bond between the same and is adapted to the irregular surfaces of the wounds, and (iii) wound healing since the fibrin adhesive promotes the fibroblast growth which in combination with a hemostasis and an efficient adhesion between the surfaces of the wound, improves wound healing. To enable performing said applications, the composition of the invention must be contacted with an aqueous medium such as has been mentioned above for a short time period, generally equal to or less than 5 minutes, preferably comprised between 1 and 3 minutes.

In a particular embodiment, the composition of the invention, comprising active ingredients, optionally lyophilisates, and a biocompatible material, as well as the fibrin adhesive provided by this invention, can be used to inhibit or stop blood loss (hemorrhage) from a wound by means of the application of said composition of the invention, or of said fibrin adhesive directly on the wound. Therefore, in another aspect, the invention relates to the use of the composition of the invention or with said fibrin adhesive provided by this invention to inhibit or stop the blood loss (hemorrhage) from a wound, or, expressed in another manner, with a composition of the invention, or with a fibrin adhesive provided by this invention for use in inhibiting or stopping the blood loss (hemorrhage) from a wound.

Likewise, in another aspect, the invention relates to a method for inhibiting or stopping the blood loss (hemorrhage) from a wound in a subject, which comprises applying the composition of the invention optionally hydrated previously or said fibrin adhesive provided by this invention on said wound. In a particular embodiment, the composition of the invention is applied directly on the wound without prior hydration, in which case, a biological fluid, for example, the blood of the subject himself can act as the aqueous medium allowing the hydration and release of the fibrinogen activator and the fibrinogen present in the composition of the invention and therefore the formation of fibrin; nevertheless, occasionally, it may be necessary and recommendable to add additional aqueous medium, for example, saline solution or saline, aqueous calcium chloride solution, Ringer's solution, sterilized water, etc., on the wound or on the composition of the invention to facilitate the hydration and interaction of the components thereof and activates the formation of fibrin. Therefore, in another particular embodiment, the composition of the invention is subjected to hydration and once hydrated it is then applied on the wound; said hydration can be carried out by conventional methods such as has been mentioned above; in this case, the body fluids (for example, blood, etc.) can act by enhancing the tissue adhesion (synergy effect) completing the final phase of the clotting process.

Once the composition of the invention, or the fibrin adhesive provided by this invention is applied on the wound, this will be kept in contact with the wound, for example, by applying a slight pressure (e.g., manually) for a sufficient time period preferably equal to or less than approximately 5 minutes, typically between approximately 10 seconds and 3 minutes so that the blood clots in the interphase between the composition of the invention, or said fibrin adhesive and the wound, the hemorrhage being stopped. In a particular embodiment, said wound has been caused by a lesion, trauma or accident, or by a surgical method.

In another particular embodiment, due to the adhesive properties of the fibrin, the composition of the invention can be used to adhere or bind the edges of a wound. Therefore, in another aspect, the invention relates to the use of the composition of the invention, or of the fibrin adhesive provided by this invention to adhere wounds or, expressed in another manner, with a composition of the invention or in a fibrin adhesive provided by this invention, for use in wound adhesion.

Likewise, in another aspect, the invention relates to a method to adhere (bind) the edges of a wound in a subject in need of treatment, which comprises applying the composition of the invention, or the fibrin adhesive provided by this invention on at least one of the edges of the wound to be bound. In a particular embodiment, the composition of the invention is applied directly without prior hydration, in which case, a biological fluid, for example, blood, vitreous humor, etc., of the subject, can act as the aqueous medium allowing the hydration and interaction of the fibrinogen activator and the fibrinogen present in the composition of the invention and therefore, the formation of the fibrin net; nevertheless, occasionally, it may be necessary to add additional aqueous medium, for example, saline solution or saline, aqueous calcium chloride solution, etc., on at least one part of the wound to be bound or on the composition of the invention to facilitate the hydration and interaction of said components and to activate the formation of the fibrin. In another particular embodiment, the composition of the invention is subjected to hydration, such as has been mentioned above, and once hydrated, it is then applied on at least one of the edges of the wound to be bound and/or the whole of the wound to be bound, the composition of the invention, or fibrin adhesive provided by this invention, will be kept in contact, for example, by applying a slight pressure (e.g., manually) for a sufficient time period, preferably equal to or less than approximately 5 minutes, typically between approximately 10 seconds and 3 minutes so that the composition adheres to the wound. In a particular embodiment, the composition of the invention or the fibrin adhesive provided by this invention can be used to bind allografts or prosthesis due to the ease of the composition of the invention to be molded and made into the shape and size desired; this application can be potentially interesting in the cases of skin allografts in burns. In another particular embodiment, the composition of the invention or the fibrin adhesive provided by this invention is used as eye bioadhesive for adhering eye tissue damaged in a traumatic or accidental manner or by lesions or as a result of an eye surgery, for example, cataract, pterygium, conjuntivochalasis, as well as in any other surgical method within any medical specialty in which it can become useful.

The composition of the invention can be contained, if desired, in a sterile, impermeable and sealed, for example, sealed in vacuum, recipient or container, which facilitates the preservation and extraction of the composition of the invention without contamination. Non-limiting illustrative examples of materials which can be used to pack or contain the composition of the invention include aluminium, plastic, glass, etc., or any other conventional primary packaging material which can be easily sterilized, isolated from humidity and which preserves the adhesive properties of the composition of the invention. The sterilization can be carried out by conventional methods, for example by gamma (γ) radiation, subjecting the composition of the invention packed or contained in said primary packaging to said radiation, or any other process of sterilization in primary packaging which do not alter the properties of the present invention.

The followings examples illustrate the invention and should not be considered in limiting sense thereof.

### EXAMPLE 1

### Producing fibrinogen and fibrinogen activator (thrombin) lyophilisates

### Materials and methods

Fibrinogen and fibrinogen activator (thrombin): The material used as source of fibrinogen and thrombin was the commercial product Tissucol^{®} Duo (Baxter), a fibrin adhesive the complete formulation of which is made up of 2 syringes preloaded with 2 mL of frozen Tissucol^{®} solution containing at most per mL:
- in one preloaded syringe, 130 mg of total protein (fibrinogen) (115 mg of coagulable protein, 50 U of Factor XIII and 120 pg of plasminogen) and 3,000 KIU (Kallidinogenase Inactivator Units) of bovine aprotinin, and excipients: human albumin, glycine, sodium chloride, sodium citrate, polysorbate 80, creatinine and sterilized water for injectable preparations; and
- in another preloaded syringe, 2 mL of frozen thrombin 500 solution, containing per mL: 500 IU of human thrombin and 40 micromoles of calcium chloride, and, as excipients: human albumin, sodium chloride, glycine and sterilized water for injectable preparations.

Lyophilization: Many conditions were assayed for the purpose of identifying the optimum temperature, time and pressure conditions for the process of lyophilizing each of the active ingredients (thrombin and fibrinogen) present in the commercial product Tissucol^{®} separately, preserving both the physicochemical and biological properties thereof.

Thrombin: Aliquots of 100-500 µL of thrombin were weight and they subsequently froze to -80°C and lyophilized in a freeze-drier under different conditions until establishing the optimum conditions described in Table 1. Once the process of lyophilization ended the samples were extracted from the equipment and the final weight of the Eppendorf was determined for the purpose of determining the content of water extracted.

Fibrinogen: Aliquots of 500 µL of fibrinogen which were diluted with MiliQ water up to three times the initial volume thereof and homogenized by means of mechanical stirring for 2 minutes were dispensed in Eppendorf tubes; they were then froze at -80°C and lyophilized in a freeze-drier under different conditions until establishing the optimum conditions described in Table 2. Once the process of lyophilization ended the samples were extracted from the equipment and the final weight of the Eppendorf was determined for the purpose of determining the content of water extracted.

According to the different tests performed, the following conditions for lyophilizing the active ingredients of the Tissucol^{®} (thrombin and fibrinogen) were established:
- Process for lyophilizing the thrombin samples: it was performed by means of a 5.5 hour program, leaving the equipment to pre-cool for 1 hour; the conditions of the program designed to lyophilize between 0.1-0.5 mL of sample are shown in Table 1.

**Table 1**

| | | | | |
|---|---|---|---|---|
| Conditions of the process for lyophilizing a thrombin sample | | | | |

| Step | Process | Time (h) | Vacuum(mbar) | Tray T° (°C) |
|---|---|---|---|---|
| 1 | Cold and vacuum | 0.5 | 0.500 | -84 |
| 2 | Heating | 3 | 0.500 | 0 |
| 3 | Heating | 2 | 0.001 | 10 |
| 4 | Final | | | |

| | | | | |
|---|---|---|---|---|
| [1 mbar: 10² Pa] | | | | |

The resulting lyophilisate ("thrombin lyophilisate") is a solid of homogenous yellowish color, very stable to the change of temperature produced during the process of loading and unloading the freeze-drier and the first step of the lyophilizing program is where the greatest drawbacks in the process of lyophilization were present.
- Process for lyophilizing the fibrinogen samples: it was performed by means of a 10.5 hour program, leaving the equipment to pre-cool for 1 hour; the conditions of the program designed to lyophilize samples of 1.5 ml (corresponding to dissolving 0.5 ml of the Tissucol^{®} fibrinogen stock solution and completed with 1.0 ml of MiliQ water) are shown in Table 2.

**Table 2**

| | | | | |
|---|---|---|---|---|
| Conditions of the process for lyophilizing a fibrinogen sample | | | | |

| Step | Process | Time (h) | Vacuum (mbar) | Tray T° (°C) |
|---|---|---|---|---|
| 1 | Cold and vacuum | 0.5 | 0.500 | -84 |
| 2 | Heating | 7 | 0.500 | -4 |
| 3 | Heating | 3 | 0.001 | 10 |
| 4 | Final | | | |

| | | | | |
|---|---|---|---|---|
| [1 mbar: 10² Pa] | | | | |

The resulting lyophilisate ("fibrinogen lyophilisate") is a solid of very homogenous whitish color and of low rehydration requiring vigorous stirring, preserving the physicochemical and biological properties thereof after reconstitution from the lyophilisate.

The processes of lyophilization described above generated stable and functional thrombin lyophilisates and fibrinogen lyophilisates, such as can be verified by means of the in vitro and in vitro *ex vivo* assays performed.

### EXAMPLE 2

### In vitro assays of the thrombin and fibrinogen lyophilisates: reconstitution and adhesion

The thrombin lyophilisates and the fibrinogen lyophilisates obtained as described in Example 1 were subjected to reconstitution tests by hydration and in vitro *ex vivo* adhesion tests.

Reconstitution test of the thrombin lyophilisate and fibrinogen lyophilisate: each of said lyophilisates was hydrated with a volume of MiliQ water corresponding to the humidity lost in each case and determined by weight loss. After adding the MiliQ water to each lyophilisate, the resulting mixture was subjected to mechanical stirring until the complete rehydration of the sample. The thrombin lyophilisate was reconstituted almost immediately after adding the MiliQ water, whereas the fibrinogen lyophilisate required a time of stirring under the assayed conditions comprised between approximately 3 and approximately 15 minutes, typically between approximately 5 and 10 minutes, commonly about approximately 7 minutes for the fibrinogen. Generally, the results obtained clearly showed that both the thrombin lyophilisate and the fibrinogen lyophilisate are completely reconstituted under the assayed conditions (mechanical stirring (manual) and room temperature) provided that it is reconstituted in the suitable and/or optimum amount of water.

*In* vitro adhesion test: To perform this test, first, the fibrinogen and thrombin lyophilisates were reconstituted in the optimum volumes of MiliQ water separately, as has been described above. Then, the resulting fibrinogen and thrombin solutions were placed on a slide, one on top of another, and they were mixed in a "thrombin solution: fibrinogen solution" ratio of 1:1 (v:v), in a total mixture volume of 100 µL. Said mixture preserved the proportion of 50 IU of thrombin per each 9 mg of fibrinogen [this ratio has been established based on the stoichiometry of the different components (thrombin and fibrinogen) present in the starting product Tissucol^{®}]. The resulting mixture was homogenized, and then a cover slip was placed above to determine the in vitro adhesive capacity of said mixture. The results obtained clearly showed that the reconstituted thrombin and fibrinogen lyophilisates, in a ratio 1:1 (v:v), in the proportion of active ingredient indicated above, has a good adhesive capacity after 2, 5, 10 and 15 minutes, the adhesive capacity thereof being increased in a manner directly proportional with the incubation temperature (20°C - 34°C) and time.

These assays (*in vitro* adhesion and reconstitution) clearly showed that the thrombin and fibrinogen lyophilisates are a solid presentation reconstituted completely and that, once combined and contacted to one another in the suitable proportions, have adhesive properties at *in vitro* level. Due to the presentation form (lyophilisate) thereof, the thrombin and fibrinogen lyophilisates can be stored throughout prolonged times (at least, up to 1 year), without losing their physicochemical and biological properties, without having to resort to preserving them at low temperatures (frozen) but typically at room temperature, which makes up a significant advantage in comparison to other similar fibrin adhesives which must be preserved at low temperatures (frozen) and with the resulting economic cost, time and equipment necessary to temper the products, prepare and activate the fibrin adhesive.

### EXAMPLE 3

### Producing fibrin adhesives in the form of films comprising thrombin and fibrinogen lyophilisates

This assay was performed for the purpose of producing solid fibrin adhesive prototypes in the form of films (sheets or laminas), from said thrombin and fibrinogen lyophilisates; said films were obtained by means of using techniques commonly used in the production of solids such as the "casting" of emulsions, the drying, the molding by compression, among others.

### Molding by compression

To perform these tests of molding by compression a hydraulic press (Collin, model P 200 E) was used. Initially, thrombin lyophilisate-based films and fibrinogen lyophilisate-based films were produced separately. To that end, 280 mg of thrombin lyophilisate and 230 mg of fibrinogen lyophilisate were placed separately or previously mixed between the metal plates of the hydraulic press on a surface of the hydraulic press coated with aluminium foil as the anti-adherent surface, and many conditions of maximum pressure [between 150.0 bars (150 x 10⁵ Pa) and 300.0 bars (300 x 10⁵ Pa)], time [between 1 and 10 minutes] and temperature [between 20°C and 200°C] were assayed. In a specific embodiment a maximum pressure of approximately 240.0 bars (240x10⁵ Pa) was applied for a time period of approximately 5 minutes and at a temperature of approximately 50°C to not exceed the denaturing temperature of the fibrinogen (60°C). The two metal plates of the hydraulic press were coated with aluminium foil before placing each of the lyophilisates (thrombin lyophilisate and fibrinogen lyophilisate) separately or the mixture, for the purpose of preventing or minimizing the adhesion thereof to the metal plates of the hydraulic press.

The results obtained clearly showed that the film formed by compression of the fibrinogen lyophilisate has a good consistency, strength, flexibility and an easy handling; contrarily and in some occasions it was observed that, upon removing the film formed by compressing the thrombin lyophilisate, the latter was more fragile being broken easily, therefore said material by itself did not have the consistency suitable for the handling thereof.

However, in view of the good consistency, strength, flexibility and handling characteristics that the film obtained from the fibrinogen lyophilisate had, an equimolar mixture of 280 mg of thrombin lyophilisate and 230 mg of fibrinogen lyophilisate was prepared (maintaining the ratio of the initial formulation present in the commercial starting product, the Tissucol^{®}, but with different w/w ratio due to the differences in the content of water which has been eliminated), was placed between the metal plates of the hydraulic press, coated with aluminium foil, and many conditions of maximum pressure [between 150.0 bars (150 x 10⁵ Pa) and 300.0 bars (300 x 10⁵ Pa)], time [between 1 and 10 minutes] and temperature [between 20°C and 200°C] were assayed. In a specific embodiment a maximum pressure of approximately 240.0 bars (240 x 10⁵ Pa) was applied for a time period of approximately 5 minutes and at a temperature of approximately 50°C, preventing exceeding 60°C. By mixing the fibrinogen lyophilisate with the thrombin lyophilisate in the suitable equimolar ratio before the process of compression, a resistant and malleable film having the best characteristics was generated with the mixture of the two components.

### Casting

Additionally, for the purpose of obtaining a film from thrombin lyophilisate with the suitable strength, the possibility of using a biocompatible material as a support material for the thrombin and fibrinogen individually, was evaluated. These films which contained a biocompatible material as a support of the thrombin were produced by means of generating emulsions from the active ingredient separately with a biocompatible material and casting.

Many biocompatible materials including the polylactic-co-glycolic acid (PLGA) and the polyvinyl alcohol (PVOH) were assayed as the biocompatible material to produce films containing thrombin and/or fibrinogen lyophilisates by means of this technique (emulsion-evaporation), finally the PLGA which, in addition to being a biocompatible and biodegradable material, has a broad history of application at biomedical level being selected.

By means of the technique ("casting") and using PLGA as biocompatible material, thrombin lyophilisate-based or fibrinogen lyophilisate-based sheets reconstituted in milli-Q water and the polymer PLGA 50:50 solubilized in any organic solvent such as chloroform, dichloromethane, etc, were prepared. Sheets formed from homogenizing a PLGA 50:50 solution with each of the active ingredients (fibrin or thrombin separately) generate slightly viscous emulsions. Said emulsions were subjected to a process of drying by any of the conventional drying methods (in the environment, stove, oven or lyophilization, among others) generating dry solids. Subsequently, said dry solids were pressed independently generating a sheet of thrombin and biocompatible material (T+PLGA) and another sheet of fibrinogen and biocompatible material (F+PLGA) which were pressed one on top of the other to generate a bilayer film (formed by two layers, each with a different active ingredient).

Table 3 summarizes the materials used and some of the films obtained using the different techniques [compression of lyophilisates and "casting" of emulsions] (see below).

**Table 3**

| | | | | |
|---|---|---|---|---|
| Materials used and laminas produced | | | | |

| Material | Support | Technique | Film | |
|---|---|---|---|---|
| T | No | Pressing | 1 | Pressed T (film) |
| F | No | Pressing | 2 | Pressed F (film) |
| T + F | No | Pressing | 3 | Pressed T + F (film) |
| T | PLGA | Casting | 4 | Pressed T with PLGA matrix (film) |
| F | PLGA | Casting | 5 | Pressed F with PLGA matrix (film) |
| T + F | PLGA | Casting | 6 | Pressed T + F with PLGA matrix (film) |

| | | | | |
|---|---|---|---|---|
| T: Thrombin lyophilisate; F: Fibrinogen lyophilisate PLGA: Poly(lactic-co-glycolic acid) | | | | |

The results obtained clearly showed that the mixture of thrombin lyophilisate and fibrinogen lyophilisate forms a film with good consistency and easy handling. Likewise, the films which contained PLGA as support had, generally, a good strength, easy handling, and a good stability over time (separation of the layers of PLGA (on one hand) and of fibrinogen and/or thrombin (on the other hand) was not observed for at least six months, and even 1 year.

Therefore, in summary, it was observed that, in terms of molding by compression, the best presentation of the fibrin adhesive prototype was the one formed by the mixture of the thrombin lyophilisate and the fibrinogen lyophilisate without support (PLGA), i.e., the film identified with the number 3 in Table 3. Said film obtained by means of the process of compression or pressing has good consistency, strength, flexibility and easy handling; furthermore, the fact that the two components are intimately bound and in the suitable proportions, reduces the total reaction time, and, both the production and application thereof is generally easier and more practical since only a product film is placed and said film exerts its adhesive effect when contacting with an aqueous medium due to the fact that, at that moment, the fibrinogen is hydrolyzed by the action of the thrombin with the resulting reticulation of the fibrin. The film formed by each of the active ingredients independently accompanied by PLGA and mixed at the end of the production process has demonstrated to be of easy handling and cutting. The adhesivity test demonstrated that the prototype has adhesive characteristics of interest to be used on the surface as well as a long-lasting seal.

A fibrin adhesive of the type mentioned above comprising a film formed by compressing a thrombin lyophilisate and a fibrinogen lyophilisate, as well as the fibrin adhesive prototypes formed with the thrombin and/or fibrinogen lyophilisates and PLGA had very good consistency, strength, flexibility and handling properties, as well as adhesion properties in in vitro *ex vivo* assays. Thus said compositions make up a new form of application of said compounds, and have many advantages, among which the followings are highlighted:
- Ease of application in comparison to those fibrin adhesives in which the active components (fibrinogen and thrombin) are separated, for example, in the form of a powder or a lyophilisate requiring a prior reconstitution, or in the form of frozen products requiring a prior tempering and dissolving of the active components, or in the form of solutions requiring the mixing thereof before their application;
- application of the suitable single-dose of the active components for each particular case, i.e., the single-dose presentation form suitable to each application are selected whereby the loss of product as occurs with the commercial fibrin systems existing currently is prevented, furthermore, the risk of cross-contamination between patients being eliminated; and
- prolonged preservation since the fibrin adhesive is activated only when the film contacts with an aqueous medium

### EXAMPLE 4

### Producing films from thrombin emulsions and from fibrinogen emulsions

### 4.1 Producing emulsions

0.280 g of thrombin lyophilisate (Example 1) were reconstituted until completing a volume of 0.3 mL of MiliQ water. Once the complete solubilization is achieved, they were combined with polylactic-co-glycolic acid (PLGA) (50:50) at different concentrations (5, 10, 25, 50 and 100 mg/mL) and volumes [Table 4]; then to mix the thrombin with the polymer PLGA, they were subjected to a process of homogenization by ultrasound for 30 seconds at 50 W in a Bandelin^{®} HD-3200 apparatus inducing the formation of the thrombin emulsion (T-PLGA).

Likewise, 0.23 g of fibrinogen lyophilisate (Example 1) were reconstituted with of MiliQ water until completing a volume of 0.3 mL of the solution, and then, they were combined with PLGA (50:50) at different concentrations (5, 10, 25, 50 and 100 mg/mL) and volumes [Table 4]; the resulting mixtures were subjected to a process of homogenization by ultrasound for 30 seconds at 50 W with a Bandelin^{®} HD-3200 homogenizer to induce the formation of the fibrinogen emulsions (F-PLGA).

### 4.2 Producing films

### 4.2.1 Producing films (sheets) of thrombin and PLGA or of fibrinogen and PLGA

From said thrombin and fibrinogen with PLGA emulsions previously obtained many films were produced by means of drying (dehydration) said emulsions. To that end, 60 µL of said thrombin and fibrinogen emulsions were deposited separately on surfaces which contained an anti-adherent material and were left to dry at room temperature throughout the night under hood until dry, in some cases, a film which contained thrombin and PLGA being generated, and, in other cases, a film which contained fibrinogen and PLGA being generated. In all the cases, the water content of said films was less than 10% by weight of the corresponding film, generally less than 5%, typically less than 3% and, commonly less than 1% by weight with respect to the total weight of the corresponding film, therefore said films were substantially water-free, i.e., they were substantially dehydrated or dry. The water content of a layer (or film) such as the one mentioned above can be determined by conventional methods, for example, assessing the water loss by weight or analytic techniques such as Karl-Fisher titration, infrared spectroscopy among others. Finally, both the dry solid comprising thrombin and biocompatible material (T-PLGA) and the dry solid comprising fibrinogen and biocompatible material (F-PLGA) are subjected individually to a process of direct pressing (or compression) until forming a sheet with each of them. Table 4 show the films produced according to the methodology described above.

**Table 4**

| | | |
|---|---|---|
| Films produced from thrombin or fibrinogen and PLGA emulsions after molding and drying | | |

| Component | Material | A.I.: PLGA |
|---|---|---|
| F + PLGA | Dried F with a PLGA-5 matrix | 100:900 |
| T + PLGA | Dried T with a PLGA-5 matrix | 100:900 |
| F + PLGA | Dried F with a PLGA-10 matrix | 100:900 |
| T + PLGA | Dried T with a PLGA-10 matrix | 100:900 |
| F + PLGA | Dried F with a PLGA-25 matrix | 100:900 |
| T + PLGA | Dried T with a PLGA-25 matrix | 100:900 |
| F + PLGA | Dried F with a PLGA-50 matrix | 100:900 |
| T + PLGA | Dried T with a PLGA-50 matrix | 100:900 |
| F + PLGA | Dried F with a PLGA-100 matrix | 100:900 |
| T + PLGA | Dried T with a PLGA-100 matrix | 100:900 |
| F + PLGA | Dried F with a PLGA-25 matrix | 300:700 |
| T + PLGA | Dried T with a PLGA-25 matrix | 300:700 |
| F + PLGA | Dried F with a PLGA-25 matrix | 600:400 |
| T + PLGA | Dried T with a PLGA-25 matrix | 600:400 |

| | | |
|---|---|---|
| F: Fibrinogen T: Thrombin A.I.: Active ingredient (F or T) PLGA: Polylactic-co-glycolic acid (PLGA) A.I.: PLGA: corresponds to the ratio (v/v) of AI:PLGA PLGA-5: 5 mg of PLGA/mL PLGA-10: 10 mg of PLGA/mL PLGA-25: 25 mg of PLGA/mL PLGA-50: 50 mg of PLGA/mL PLGA-100: 100 mg of PLGA/mL | | |

### 4.2.2 Producing bilayer films of thrombin, fibrinogen and PLGA

Additionally, from the solid sheets of thrombin and fibrinogen obtained above, films which contained both thrombin and fibrinogen in bilaminar or bilayer arrangement were produced for the subsequent evaluation thereof as fibrin adhesive. To that end, the protocol mentioned below was followed.

Briefly, the sheets of thrombin and biocompatible material (T+PLGA) and of fibrinogen and biocompatible material (F+PLGA) obtained separately above were pressed individually to reduce its thickness and are subsequently contacted (deposited one on top of the other) and pressed for a second and last time one on top of the other to generate a film bilayer (formed by two layers with different active ingredient in each of them), placing them between the metal plates of a hydraulic press coated with aluminium foil (as anti-adherent material), and many conditions of maximum pressure [between 150.0 bars (150 x 10⁵ Pa) and 300.0 bars (300 x 10⁵ Pa)], time [between 1 and 10 minutes] and temperature [between 20°C and 200°C] were assayed. In a specific embodiment, a maximum pressure of approximately 240.0 bars (240 x 10⁵ Pa) was applied for a time period of approximately 5 minutes and at a temperature of approximately 50°C, preventing exceeding 60°C. and generating a single film formed by 2 layers, one of T+PLGA and another of F+PLGA, the water content of which was equal to or less than 10% by weight with respect to the total weight of the film.

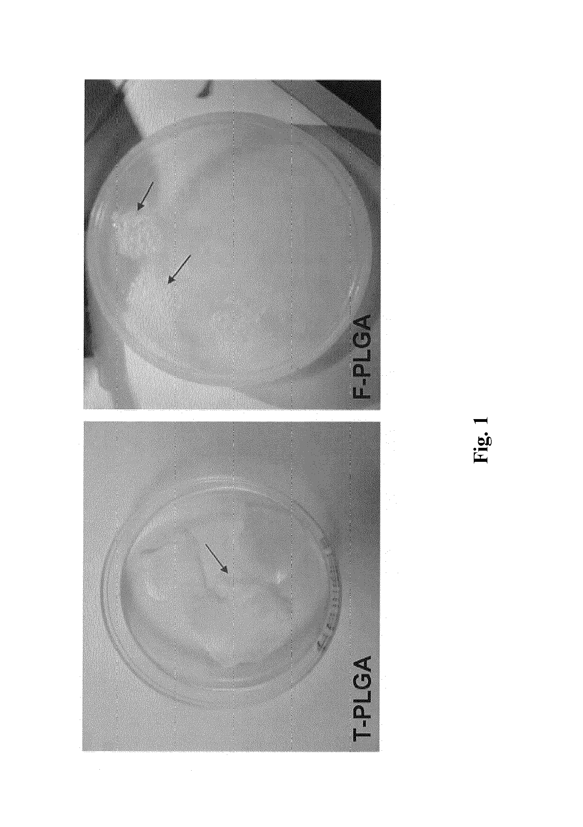

The thrombin and PLGA or fibrinogen-PLGA emulsions obtained in both cases were whitish and pearlescent fluids, respectively, of easy handling, stable for relatively long time periods (about 10 hours) and formed a manipulable solid. The formation of the bilayer (two layers) film was very simple allowing obtaining, after the process of co-pressing a film with two layers, a first layer of thrombin and PLGA and a second layer of fibrinogen and PLGA, which had optimum manipulation conditions, good aspect of the film obtained and ease of producing a single bilayer film [Figure 1, T-PLGA+F-PLGA]. Likewise, it was observed that the ratio of the emulsions formed with each active ingredient [thrombin and PLGA (T-PLGA) and fibrinogen and PLGA (F-PLGA)] of 400:600 (v:v) was the optimum ratio in terms of production (based on the stoichiometry of the formulation of the starting product, Tissucol^{®}).

### EXAMPLE 5

### Adhesion assays of fibrin adhesive prototypes

For the purpose of evaluating the adhesion capacity of the different fibrin adhesive prototypes obtained according to the teachings of the present invention in vitr*o* and *in vitro ex vivo* adhesions assays such as described below, were performed. To perform said assays, those fibrin adhesive prototypes (films) developed by this invention which had a good stability and an easy handling, characteristics which in addition to a suitable adhesion capacity, would require a fibrin adhesive developed according to the teachings of the present invention, were initially selected. In this sense, the fibrin adhesive prototypes assayed and with relevant results were the following:
- Fibrin adhesive prototypes obtained by compressing the thrombin (T) and fibrinogen (F) lyophilisates; and
- Fibrin adhesive prototypes obtained by drying and molding emulsions prepared from the thrombin (T) and fibrinogen (F) lyophilisates reconstituted with MiliQ water and emulsified with PLGA.

### In vitro adhesion test

In the case of the fibrin adhesive prototypes prepared by compressing thrombin and fibrinogen lyophilisates, the *in vitro* adhesion tests were performed in a manner as described in Example 2, i.e., suitable equimolar amounts of compressed thrombin and fibrinogen lyophilisates were deposited on a cover slip and were hydrated with between, 30 and 60 ml of MiliQ water viscous solutions being generated. Volumes 1:1 of the thrombin and fibrinogen solutions obtained were then mixed on a slide (said volumes provided the thrombin activity and the concentration of fibrinogen suitable so that the fibrin was generated and the adhesion capacity thereof could be evaluated), homogenizing the resulting mixture. Subsequently it was placed above a cover slip, incubating at room temperature and at 34°C individually for a time period of approximately 2 minutes, and determining the adhesion of the cover slip to the slide, which is indicative of the in vitro adhesive capacity of the mixture assayed. The adhesion can be verified by conventional methods, for example, by means of tensile strength under strain test of the cover slip adhered to the slide to determine the force which must be exerted to separate the cover slip per unit of surface, or by means of tensile strength under shearing test; nevertheless, in this case, it was empirically verified by observing whether or not the cover slip is peeled off from the slide (Table 5).

In the case of the fibrin adhesive prototypes prepared by compressing (composition 2) and drying thrombin and PLGA (T-PLGA) emulsions and fibrinogen and PLGA (F-PLGA) emulsions, to generate the films the *in vitro* adhesion tests were performed in a manner as has been described above; briefly, a 0.3 x 0.4 cm² fragment of the fibrin and thrombin film of each composition (which contained the thrombin activity and the concentration of fibrinogen suitable so that the fibrin would be produced and the adhesion capacity thereof could be evaluated) was deposited on a slide. A volume of approximately 70-100 µL of milli-Q water was then added, and after a time of hydration of the prototype (between 1-3 minutes), a cover slip was placed above and was incubated at room temperature and at 34°C for 5 minutes. Similarly to the case above, in this case, the adhesion was empirically verified by observing whether or not the cover slip was peeled off from the slide (Table 5).

**Table 5**

| | | | |
|---|---|---|---|
| *In vitro* adhesion tests with the different films produced | | | |

| Composition | Production | Film F:T Ratio (w/w) | Adhesivity |
|---|---|---|---|
| F and T Lyophilisate | Compression | 1:1 | (+) |
| | | 4:2 | (-) |
| | | 2.6:1 | (+) |
| | | 5.4:2 | (-) |
| F + PLGA+ T + PLGA | Emulsion, drying and compression | 1:1 | (+) |

| | | | |
|---|---|---|---|
| (+) :Adhesion present; (-)Adhesion not present. | | | |

### In vitro ex vivo adhesion tests

These tests were performed to evaluate the tissue adhesion capacity of the different fibrin adhesive prototypes obtained according to the teachings of the present invention in tempered enucleated pig eyes at 34°C in incubator to thus maintain the tissues at a temperature similar to the temperature of the *in vivo* cornea [Mapstone, R., Normal thermal patterns in cornea and periorbital skin. Br J Ophthalmol, 1968. 52(11):818-27; Moller-Pedersen, T., et al., Evaluation of potential organ culture media for eye banking using human donor corneas. Br J Ophthalmol, 2001. 85(9):1075-9; Tanelian, D.L. and R.W. Beuerman, Recovery of corneal sensation following hard contact lens wear and the implication for adaptation. Invest Ophthalmol Vis Sci, 1980. 19(11) :1391-4]. Incisions in the pig eyes similar to the incisions performed in cataract surgery were performed.

To that end, a cataract surgery was performed in enucleated and tempered pig eyes such that a main incision and a service incision were made on the entire eyes with a 2.75 mm slit knife. The fibrin adhesive prototypes (films) assayed were cut according to the dimensions and shape of the surgical cut, (approximately 0.3 x 0.4 cm²) and 50% were introduced in the wounds and the remaining 50% on the surface of the tissue, or deposited on the wound surface binding the two surfaces of the wound, side by side of the incision. After applying the fibrin adhesive prototype to be assayed, the treated eyes were incubated at different temperatures simulating the physiological conditions (between 34°C and 37°C) for different time periods (between 5 minutes and 1/2 hour, since the time to produce a bioadhesive effect in operating theatre must not be very long), and observations at the different times were performed for the purpose of evaluating the adhesive capacity of the different prototypes assayed. To that end a drop of fluorescein was applied on the surface of the treated eye and saline was injected through the service incision to view the possible exit of the saline through the incision covered or sealed with the fibrin bioadhesive evaluated by means of washing the fluorescein in the event that there was no adhesion, or not washing the fluorescein in the event that there was tissue adhesion.

Likewise, the sealing capacity of each prototype, "sealing" being defined as the capacity of a prototype to prevent the exit of fluid (saline) through the incision in which the prototype is evaluated, was determined. The "adhesion" defined as the strength of the prototype evaluated to be removed from the tissue and to keep the two sides of the wound bound to one another was also evaluated. Additionally, the "removal" capacity of a fibrin adhesive prototype defined as the ease or difficulty of the prototype to be removed from the incision as well as the ease thereof to handle the same was also evaluated.

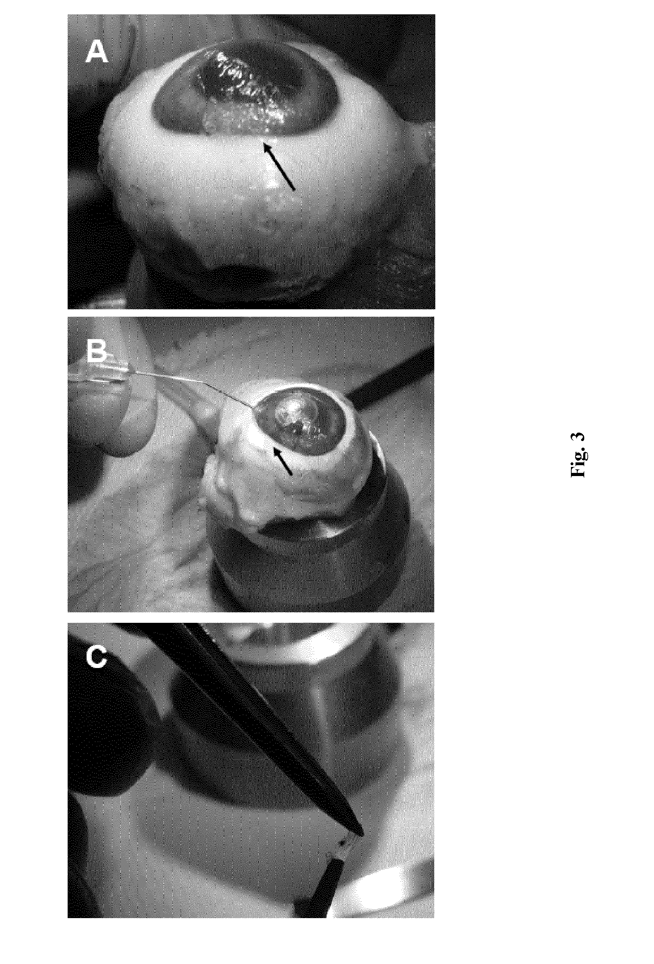

Figure 3 and Table 6 show the results of the adhesive capacity of some fibrin adhesive prototypes developed. As can be seen, two of the prototypes developed offered very favorable results with respect to the adhesive activity thereof at times less than 15 minutes of the assay.

**Table 6**

| | | | | | | |
|---|---|---|---|---|---|---|
| Results of the *in vitro ex vivo* assays of some prototypes | | | | | | |

| Sample | Location | t_{T} (min) | Sealing | Adhesion | Removal | Observations |
|---|---|---|---|---|---|---|
| F+T pressing | Surface | 15 | Yes | Yes | Easy | +++ |
| T-PLGA + F-PLGA | Surface | 8 | Yes | Yes | Easy | +++ |

| | | | | | | |
|---|---|---|---|---|---|---|
| t_{T}: Total time of incubation at 34°C. "Sealing": Relates to the capacity of the prototype to prevent the exit of fluid (saline) through the incision in which the prototype is evaluated. "Adhesion": Relates to the strength of the prototype evaluated to be removed from the tissue and to keep the two sides of the wound bound to one another. "Removal": Relates to the ease or difficulty of the prototype to be removed from the incision as well as the ease thereof to handle the same. "Observations": Refer the general rating of the prototype assayed as bioadhesive, being evaluated at qualitative level with respect to ease of handling, sealing, adhesion, malleability among other characteristics. A single "+" sign indicates that the fibrin adhesive prototype assayed had minimum bioadhesive properties, whereas 3 "+" ("+++") signs indicates that the fibrin adhesive prototype assayed has the best bioadhesive characteristics. F+T pressing: Film obtained by compressing a mixture of fibrinogen lyophilisate and thrombin lyophilisate. T-PLGA + F-PLGA: Film obtained by molding and drying a thrombin and PLGA emulsion and a fibrinogen and PLGA emulsion. | | | | | | |

As can be seen in said Table 6, the fibrin adhesive prototype obtained by compressing a mixture of thrombin lyophilisate and fibrinogen lyophilisate in the suitable amounts (F+T pressing), once reconstituted by contact with an aqueous medium, preserves the adhesive properties thereof, is of easy handling and shows adhesive properties at 15 minutes by surface application on the incision.

Likewise, the fibrin adhesive prototype obtained by drying and compressing a thrombin and PLGA emulsion and by drying and molding a fibrinogen and PLGA emulsion (T-PLGA + F-PLGA), showed, in addition to a good stability and ease of handling, ease of cutting, as well as adhesive properties at 8 minutes of incubation when it is applied on the surface; it also had a long-lasting sealing, very interesting characteristics at surgical level.

In view of the results obtained in the *in vitro* and *in vitro ex vivo* assays 2 fibrin adhesive prototypes with ideal characteristics for application as fibrin adhesive, advantageously, in the form of single-dose film, particularly, at ophthalmology level and more specifically, on surgical processes of the surface eye and possible applications in other type of wounds or incisions have been identified; said fibrin adhesive prototypes are:
- the prototype identified as "F+T pressing", obtained by compressing a mixture of a thrombin lyophilisate and a fibrinogen lyophilisate with the proportion thrombin activity and concentration of fibrinogen suitable; and
- the prototype identified as "T-PLGA + F-PLGA", obtained by means of individual drying of a thrombin and PLGA emulsion (T-PLGA) and another fibrinogen and PLGA emulsion (F-PLGA), yielding a solid whereby a layer is generated by means of compressing each of them, and finally, the layer obtained containing the fibrinogen is placed on the layer containing thrombin and are subjected to a final process of compaction by means of pressing producing a bilayer film.

## Claims

1. A composition in the form of film comprising:
a) a layer (A) comprising a fibrinogen activator and a biocompatible material; and
b) a layer (B) comprising fibrinogen and a biocompatible material;
wherein the fibrinogen activator and the fibrinogen are not present simultaneously in one and the same layer.

2. The composition according to claim 1, wherein said fibrinogen activator is a thrombin.

3. The composition according to claim 1, wherein said layer (A) further comprises calcium ions, and/or said layer (B) further comprises a product selected from factor XIII, aprotinin, plasminogen, and mixtures thereof.

4. The composition according to claim 1, wherein said biocompatible material is a poly(lactic-co-glycolic) acid (PLGA) copolymer.

5. A method for obtaining a composition in the form of film according to claim 1, which comprises:
i) depositing an emulsion comprising a fibrinogen activator and a biocompatible material on a surface comprising an anti-adherent material, drying and pressing the solid generated until forming a sheet [layer (A)];
ii) depositing an emulsion comprising fibrinogen and a biocompatible material on a surface comprising an anti-adherent material, drying and pressing the solid generated until forming a sheet [layer (B)];
iii) depositing the sheet comprising fibrinogen and a biocompatible material [layer (B)] on the sheet comprising a fibrinogen activator and a biocompatible material [layer (A)];
or, alternatively,
depositing the sheet comprising a fibrinogen activator and a biocompatible material [layer (A)] on the sheet comprising fibrinogen and a biocompatible material [layer (B)]; and
iv) subjecting the product of step iii) to a compression process until compacting the two layers and obtaining said composition in the form of film comprising said layers (A) and (B) and wherein the a fibrinogen activator and the fibrinogen are not present simultaneously in one and the same layer.

6. A composition in the form of film comprising a compressed mixture of a lyophilisate comprising a fibrinogen activator and a lyophilisate comprising fibrinogen.

7. A composition in the form of film comprising a fibrinogen activator and fibrinogen obtained by compressing a mixture comprising (i) a lyophilisate comprising a fibrinogen activator and (ii) a lyophilisate comprising fibrinogen.

8. The composition according to any of claims 6 or 7, wherein said fibrinogen activator is a thrombin.

9. The composition according to any of claims 6 or 7, further comprising calcium ions and/or a product selected from factor XIII, aprotinin, plasminogen, and mixtures thereof.

10. A method for obtaining a composition in the form of film according to claim 7, which comprises compressing a mixture comprising (i) a lyophilisate comprising a fibrinogen activator and (ii) a lyophilisate comprising fibrinogen.

11. A fibrin adhesive comprising a hydrated composition in the form of film according to any of claims 1 to 4 or 6 to 9 or obtainable by means of a method according to claim 5 or 10.

12. A composition according to any of claims 1 to 4 or according to any of claims 6 to 9 or a fibrin adhesive according to claim 11 for use in inhibiting or stopping blood loss from a wound.

13. A composition according to any of claims 1 to 4 or according to any of claims 6 to 9 or a fibrin adhesive according to claim 11 for use in adhering tissues.

14. The composition for use according to claim 13 in adhering damaged eye tissue.

15. The composition for use according to claim 13, wherein the eye tissue has been damaged in a traumatic or accidental manner by a lesion or as a result of an eye surgery.
